# EUROPÄISCHE PATENTANMELDUNG

(11) **EP 3 736 023 A1**
(43) Veröffentlichungstag der Anmeldung: **11.11.2020**
(21) Anmeldenummer: 19173781.6
(22) Anmeldetag: 10.05.2019
(51) Int. Cl.: A61Q 11/00, A61K 8/25, A61K 8/73, A61K 8/60, A61K 8/81, A61C 17/16

(54) **THIXOTROPE ZAHNPFLEGEZUSAMMENSETZUNG, SYSTEM BESTEHEND AUS DER ZAHNPFLEGEZUSAMMENSETZUNG UND EINEM SPENDER SOWIE VERWENDUNG DER ZAHNPFLEGEZUSAMMENSETZUNG IN EINEM VERFAHREN ZUM GLEICHZEITIGEN REINIGEN MEHRERER, VORZUGSWEISE ALLER ZÄHNE**

(71) Anmelder: BLBR GmbH, 82031 Grünwald (DE); Aerochemica GmbH, 47906 Kempen (DE)
(72) Erfinder: KEINER, Michael, 35619 Braunfels (DE); KILDERS, Claudia, 46519 Alpen (DE); Dr. BRUDER, Gregor, 47608 Geldern (DE)
(74) Vertreter: Becker & Kurig Partnerschaft Patentanwälte PartmbB

(57) **Zusammenfassung**

Die vorliegende Erfindung betrifft thixotrope Zahnpflegezusammensetzungen und deren Verwendung zur Reinigung und Pflege der Zähne und des Mundes. Durch die Bereitstellung einer thixotropen Matrix werden im unbewegt lagernden Produkt die Putzkörper der Zusammensetzung stabil in Schwebe gehalten und deren Absinken wird verhindert. Mit der Zubereitung in Schaum- oder Aerosolform kann die Zusammensetzung gleichmäßig, schnell und effizient auf Mundeinsatzelemente, Reinigungsprothesen und sonstige Vorrichtungen, die zum gleichzeitigen Reinigen mehrerer oder aller Zähne dienen, aufgetragen werden. Die Handhabung wird wesentlich vereinfacht.

## Beschreibung

### GEBIET DER ERFINDUNG

Die vorliegende Erfindung betrifft eine thixotrope Mund-, Zahnpflege- und Zahnreinigungszusammensetzung, welche zur Anwendung bei der Zahnreinigung und -pflege mittels einer Zahnputzvorrichtung, insbesondere einer elektrischen Schall- bzw. Ultraschallzahnbürste, für die gleichzeitige Reinigung mehrerer, vorzugsweise aller Zähne eines Benutzers, geeignet ist. Durch die Einbettung der Putzkörper in eine thixotrope, stabile Matrix werden diese im unbewegt lagernden Produkt stabil in Schwebe gehalten und ein Absinken der Putzkörper verhindert, während das bewegte Produkt beim Applikationsvorgang auf den Mundeinsatz einer Zahnputzvorrichtung, bedingt durch den Thixotropieeffekt, dann bei niedriger Viskosität beispielsweise als Schaum ausgebracht werden kann. Die vorliegende Erfindung betrifft weiterhin die Verwendung der thixotropen Zusammensetzung in einem Verfahren zur gleichzeitigen Reinigung mehrere, vorzugsweise aller Zähne mittels der Zahnputzvorrichtung.

### STAND DER TECHNIK

Generell liegen Mund- und Zahnpflegemittel in Pasten- oder Cremeform vor. Sie haben eine Viskosität von 60.000 bis 80.000 mPas (Millipascalsekunden; gemessen bei 20 °C) und werden aus Tuben oder Standbehältern durch manuelles Zusammendrücken des Behälters auf den Bürstenkopf einer Zahnbürste abgegeben. Die übliche Anwendungsmenge liegt bei 1 bis 2 ml pro Benutzung. In selteneren Fällen liegt das Mund- und Zahnpflegemittel in Gelform mit einer Viskosität von 10.000 bis 70.000 mPas (bei 20°C) vor. Auch hier wird eine Menge von 1 bis 2 ml durch Druck auf den Spenderbehälter auf den Bürstenkopf einer Zahnbürste appliziert. Durch die hohe Viskosität der herkömmlichen Zahnreinigungszusammensetzungen wird ein Absinken der Putzkörper auf den Boden des Produktbehälters verhindert.

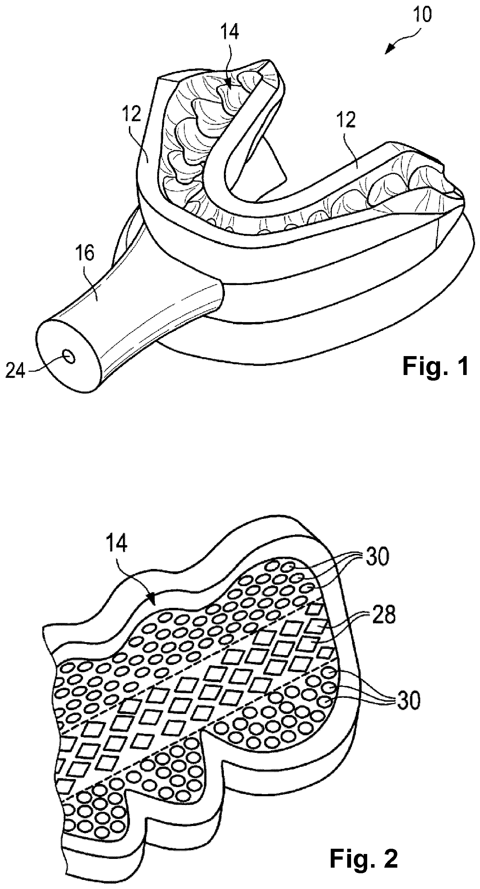

Die vorliegende Erfindung ist jedoch nicht vornehmlich auf die Anwendung der Mund- und Zahnpflege- und -reinigungszusammensetzung mit einer herkömmlichen Handzahnbürste oder einer üblichen elektrischen Zahnbürste gerichtet. Vielmehr gibt es neuere Entwicklungen auf dem Gebiet der Zahnreinigung mittels eines Mundeinsatzes oder mehrerer Mundeinsätze, die zum gleichzeitigen Reinigen mehrerer oder aller Zähne dienen. Diese Mundeinsätze (siehe z.B. Fig. 1) sind zumindest grob, vorzugsweise individuell, an die Gebissform eines Benutzers angepasst und in der Lage, durch einen Motor zu Vibrationen angeregt, mehrere oder gar alle Zähne gleichzeitig zu reinigen. Entsprechende Zahnreinigungssysteme sind etwa in DE 10 2015 109891 A1, WO 2009/137671 A1 oder WO 2015/003681 A1 offenbart. Im Sinne der vorliegenden Erfindung bedeutet die gleichzeitige Reinigung mehrerer Zähne, dass der Mundeinsatz so ausgebildet ist, dass vorzugsweise gleichzeitig mindestens 3, noch mehr bevorzugt mindestens 5, noch mehr bevorzugt mindestens 7 Zähne gereinigt werden können. Ganz besonders bevorzugt ist der bzw. sind die Mundeinsätze so ausgebildet, dass gleichzeitig alle Zähne des Oberkiefers und/oder des Unterkiefers gereinigt werden können. Gleichzeitig bedeutet, dass der oder die Mundeinsätze zu einem Zeitpunkt T1 mit allen zu reinigenden Zähnen in Kontakt stehen und diese reinigen. Im Unterschied dazu tritt der Bürstenkopf einer herkömmlichen manuellen oder elektrischen Zahnbüste zeitlich nacheinander mit den zu reinigenden Zähnen in Kontakt.

Für die Benutzung in solchen Mundeinsätzen sind die vorhandenen Darreichungsformen von Zahnreinigungs- und -pflegeprodukten, wie beispielsweise Zahnpasten, Zahncremes oder Zahngele, ungeeignet. Die hochviskosen Reinigungs- und Pflegemittel gemäß dem Stand der Technik müssten mit einer Bürste oder einem Spatel auf den Reinigungsflächen der Mundeinsätze verteilt werden, wobei die hohe Viskosität herkömmlicher Zahnpasten, Zahncremes oder -gele eine gleichmäßige Verteilung nahezu unmöglich macht. Gleichzeitig würde die Menge der zu verwendenden Creme, Paste oder des Gels auf ca. 20 bis 25 ml ansteigen, was zu einer übermäßigen Verschwendung von Ressourcen und einer Überdosierung führen würde.

Eine Lösung dieses Problems könnte die Bereitstellung einer niedrigviskoseren Zahncreme darstellen, um so eine gleichmäßige Verteilung der Putzkörper und der Wirkstoffe auf dem Mundeinsatz zu gewährleisten. Allerdings stößt die Entwicklung solcher Zusammensetzungen beispielsweise dadurch an Grenzen, dass sich in solchen niederviskosen Zusammensetzungen die Putzkörper aufgrund ihrer höheren Dichte am Boden der Produktbehälter absetzen können und dann nur noch schwer oder gar nicht mehr in der Zusammensetzung dispergiert werden können.

Es besteht daher Bedarf an neuartigen Zahnreinigungszusammensetzungen, welche zur Anwendung mit Zahnputzvorrichtungen zur gleichzeitigen Reinigung aller Zähne eines Benutzers geeignet sind. Die Zusammensetzungen müssen einfach und gleichmäßig auf entsprechenden Mundeinsätzen zu applizieren sein und dort so ausreichend und stabil haften, dass sie nicht aus den Mundeinsätzen herauslaufen, bevor diese in den Mund eingeführt wurden. Darüber hinaus müssen die Formulierungen eine stabile Dispersion der Putzkörper in der Zusammensetzung gewährleisten. Nur so ist gewährleistet, dass diese gleichmäßig in den Mundeinsätzen verteilt und alle Zähne schonend und effizient gereinigt werden können. Auch müssen die Zusammensetzungen eine zufriedenstellende Schaumentwicklung im Mund zeigen, sowie die sonstigen Anforderungen erfüllen, die an herkömmliche Zahnpasten gestellt werden.

Die vorstehend genannten Probleme wurden durch die vorliegende Erfindung gelöst.

### BESCHREIBUNG DER ERFINDUNG

Die vorliegende Erfindung betrifft gemäß einem ersten Aspekt eine thixotrope Zahnpflege- und Zahnreinigungszusammensetzung. Die erfindungsgemäße Zahnpflege-und Zahnreinigungszusammensetzung umfasst
a) 0,5 bis 30 Gew.-%, vorzugsweise 1 bis 30 Gew.-%, Putzkörper,
b) 0,5 bis 5 Gew.-% wenigstens eines Schichtsilikats,
c) 0,1 bis 2 Gew.-% wenigstens eines pyrogenen, vorzugsweise eines hydrophoben pyrogenen, Silikats,
d) 0,2 bis 5 Gew.-% wenigstens einer oberflächenaktiven Substanz, und
e) ein Lösungsmittel, vorzugsweise Wasser, ad 100 Gew.-%.

Der Putzkörper ist vorzugsweise in eine thixotrope, stabile Matrix umfassend die Komponenten b) bis e) eingebettet. Durch diese Matrix kann die Zusammensetzung in ihrem thixotropen Verhalten vorteilhaft eingestellt werden. Thixotropie bezeichnet eine zeitabhängige Änderung der Fließeigenschaften von Fluiden, bei der die Viskosität infolge andauernder äußerer Einflüsse ab- oder zunimmt und erst nach beendigter Beanspruchung wieder in die Ausgangsviskosität zurückkehrt. Damit wird die Zusammensetzung mit der Dauer ihrer Deformation dünnflüssiger. Somit wird sichergestellt, dass bei geringer mechanischer Belastung, also beispielsweise während der Lagerung, die Viskosität der Zusammensetzung hoch genug ist, um ein Absetzen von Feststoffen, beispielsweise der Putzkörper, zu verhindern, während die Viskosität bei hoher mechanischer Belastung, also beispielsweise beim Austritt der Zusammensetzung aus einer Spendervorrichtung, gering sein soll, um so einwandfrei aus der Spendervorrichtung austreten zu können. Sobald sich die Zusammensetzung auf dem Mundeinsatz befindet, also die hohe mechanische Belastung wieder wegfällt, soll sich aber wieder die höhere Viskosität einstellen, um ein Ablaufen zu verhindern.

Der Putzkörper, der auch als Abrasivmittel oder Poliermittel bezeichnet werden kann, ist im Grunde ein Schleifmittel und ermöglicht eine effektive Entfernung von Belägen beim Zähneputzen. Als Putzkörper werden in der Regel wasserunlösliche anorganische Stoffe eingesetzt, die den Zahnbeleg mechanisch entfernen, ohne den Zahnschmelz oder das Dentin zu schädigen.

In den erfindungsgemäßen Zusammensetzungen kann der Putzkörper eine Substanz oder eine Mischung von verschiedenen Substanzen enthalten. Als Putzkörper eignen sich prinzipiell alle für Zahnpflegezusammensetzungen bekannten Putzkörper. Bevorzugt geeignete Putzkörper sind Silikate oder eine Mischung von mehreren Silikaten. Das oder die Silikate können auch in Kombination mit anderen Putzkörpern eingesetzt werden.

Die erfindungsgemäßen Zusammensetzungen enthalten als Putzkörper vorzugsweise wenigstens eine Verbindung aus der Gruppe der Silikate. Silikate können als Gele oder Sole vorliegen. Eine besonders geeignete Gruppe von Silikat-Putzkörpern sind jedoch solche Silikate, die im einschlägigen Stand der Technik als gefällte Silikate ("hydrated Silica") bezeichnet werden. Gefällte Silikate sind unter anderem deshalb bevorzugt, weil sie bei ihrer Herstellung in ihren Eigenschaften, wie beispielsweise ihre Härte und ihrer verdickenden Eigenschaften, breit variiert werden können. Die Härte hat einen Einfluss auf die Abrasivität der Partikel. Ein weiterer Vorteil von gefällten Silikaten ist, dass sie gut mit Fluorid-Wirkstoffen verträglich sind. Geeignete Silikate, vorzugsweise gefällte Silikate, sind im Handel erhältlich und dem Fachmann bekannt.

Die gefällten Silikate weisen bevorzugt eine mittlere Teilchengröße im Bereich von 1 bis 50 µm, noch mehr bevorzugt von 4 bis 25 µm, höchst vorzugsweise von 6 bis 14 µm. Die mittlere Teilchengröße meint den mittels Laserbeugung gemäß ISO 13320 bestimmten d50-Wert. Diese gefällten Silikate weisen besonders bevorzugt gerundete Ecken und Kanten auf.

Weiterhin weisen die gefällten Silikate bevorzugt eine Einlehner Abrasion im Bereich von kleiner 2,0 bis 30 mg Verlust / 100 000 Umdrehungen auf. Ein erfindungsgemäß besonders geeignetes gefälltes Silikat weist eine mittlere Teilchengröße im Bereich von 5 bis 12 µm und/oder eine Einlehner Abrasion im Bereich von 2,5 bis 20 mg Verlust / 100 000 Umdrehungen auf.

Erfindungsgemäß besonders bevorzugte gefällte Silikate sind unter den Handelsbezeichnungen ZEODENT®, beispielsweise ZEODENT®124, Sorbosil®, beispielsweise Sorbosil® AC77, und Sident®, beispielsweise Sident® 8, bekannt und erhältlich.

Neben diesen sind im Handel weitere gefällte Silikate erhältlich, die sich in ihrer mittleren Teilchengrößte und/oder in ihrer Abrasivität unterscheiden. Beispielhaft, aber nicht abschließend genannt seien ZEODENT®103, ZEODENT®115, ZEODENT®116, ZEODENT®165, ZEODENT®167, Sorbosil® AC33, Sorbosil® AC35, Sorbosil® AC43, Sorbosil® AC39, Sorbosil® TC15 und Sident® 12DS. Im Umfang der vorliegenden Erfindung sind ebenso beliebige Mischungen von diesen beispielhaft genannten oder anderen gefällten Silikaten umfasst. Ein Ziel des Einsatzes einer Mischung von gefällten Silikaten ist es, einen mittleren bis hohen Abrasionsfaktor der Zahnpflege- und Zahnreinigungszusammensetzung bereitzustellen. Durch die geeignete Auswahl der gefällten Silikate können die PCR-Werte (PCR = Pellicle Cleaning Ratio) verbessert und die gewünschten RDA (RDA = radioaktiver Dentinabrieb) beibehalten werden.

Weiterhin kann der Putzkörper der erfindungsgemäßen Zusammensetzung, anstelle der gefällten Silikate oder in Kombination mit einem oder mehreren gefällten Silikaten, vorzugsweise der vorstehend genannten gefällten Silikate, mindestens eine der folgenden Substanzen umfassen. Silikate, wie beispielsweise verschiedene Aluminiumsilikate und Zirkoniumsilikate, aber auch Natrium-Aluminiumsilikate, wie beispielsweise synthetische Zeolithe, und Bentonite. Ebenso denkbar sind organische Polymere, Silica Dimethyl Silylate, Aluminiumoxid, Aluminiumhydroxid, Calciumcarbonat, Calciumpyrophosphat, Dicalciumphosphatdihydrat, Titandioxid. Diese Substanzen können einzeln oder in beliebigen Mischungen davon vorliegen. Weitere, im Stand der Technik bekannte Putzkörpersubstanzen können zusätzlich in diesem bevorzugten Putzkörper umfasst sein. Vorzugsweise beträgt der Anteil der Putzkörper, die keine gefällten Silikate sind, an der Gesamtzusammensetzung höchstens 10 Gew.-%, noch mehr bevorzugt höchstens 5 Gew.-%. Ihr Anteil beträgt also vorzugsweise 0-10 Gew.-%, noch mehr bevorzugt 0-5 Gew.-%.

Ein Ziel des Einsatzes von Putzkörpersubstanzen, wie beispielsweise von gefällten Silikaten, oder einer beliebigen Mischung von Putzkörpersubstanzen, wie beispielsweise einer Mischung von mehreren gefällten Silikaten oder einer Mischung von einem oder mehreren gefällten Silikaten mit einem oder mehreren der vorstehend genannten weiteren Putzkörpersubstanzen oder einer Mischung der vorstehend genannten weiteren Putzkörpersubstanzen, ist es einen mittleren bis hohen Abrasionsfaktor der Zahnpflege-und Zahnreinigungszusammensetzung bereitzustellen. Der Abrasionsfaktor der Zusammensetzung wird vorzugsweise als RDA-Wert der Zusammensetzung angegeben. "RDA" steht für radioaktiver Dentinabrieb. Der RDA-Wert der erfindungsgemäßen Zusammensetzungen liegt bevorzugt in einem Bereich von 30 bis 160. Bei einem RDA-Wert von weniger als 30 sind die Reinigungseffekte der Zusammensetzung geringer, während bei einem RDA-Wert von mehr als 160 die Gefahr besteht, dass die Zusammensetzung so abrasiv ist, dass sie das Zahnbein entlang der Zahnfleischlinie beschädigen kann. Besonders bevorzugt liegt der RDA-Wert der erfindungsgemäßen Zusammensetzung in einem Bereich von 50 bis 130. In diesem Bereich ist die erzielte schonende Reinigungswirkung besonders vorteilhaft.

Der Abrasionsfaktor und insbesondere der RDA-Wert einer Zahnreinigungszusammensetzung hängt sowohl von der Abrasivität des verwendeten Putzkörpers bzw. der verwendeten Putzkörpersubstanzen als auch von dessen/deren Konzentration in der Zusammensetzung ab. Deshalb wird der Putzkörper bzw. werden die Putzkörpersubstanzen, vorzugsweise das/die gefällte(n) Silikat(e), in einer ausreichenden Menge und gegebenenfalls in einem geeigneten Mischungsverhältnis zugesetzt, um den gewünschten Abrasionsfaktor einzustellen.

Der Putzkörper, vorzugsweise das wenigstens eine gefällte Silikat oder eine Mischung von mehreren gefällten Silikaten, ist mit einem Anteil von 0,5 bis 30 Gew.-%, bezogen auf die Gesamtzusammensetzung, in der erfindungsgemäßen Zusammensetzung umfasst. Vorzugsweise beträgt der Anteil des wenigstens einen Putzkörpers, vorzugsweise das wenigstens einen gefällten Silikats, 1 bis 20 Gew.-%, noch mehr bevorzugt 2 bis 15 Gew.-%.

Gemäß einer Ausführungsform enthält die erfindungsgemäße Zusammensetzung ein Gemisch aus mindestens 2, vorzugsweise 2 bis 4 der vorstehend im Detail beschriebenen gefällten Silikate. Noch mehr bevorzugt enthält das Gemisch drei gefällte Silikate. Das Gewichtsverhältnis der drei gefällten Silikate ist vorzugsweise im Bereich von 2-15 Teilen eines ersten Silikats, 1-8 Teilen eines zweiten Silikats und 0,5-5 Teilen eines dritten Silikats.

In einer solchen Mischung von zwei oder mehr gefällten Silikaten können auch solche gefällten Silikate eingesetzt werden, die sich besonders durch ihre verdickenden Eigenschaften auszeichnen, und mit solchen gefällten Silikaten kombiniert werden, die in ihrer abrasiven Eigenschaft besonders vorteilhaft sind. Die in der erfindungsgemäßen Zusammensetzung umfassten gefällten Silikate fungieren also nicht nur als Putzkörper, sondern können darüber hinaus zum Teil auch verdickende Eigenschaften haben.

Gefällte Silikate mit verdickenden Eigenschaften zeichnen sich vorzugsweise durch eine mittlere Teilchengröße in einem Bereich von mehr als 12 bis 20 µm, noch mehr bevorzugt bis maximal 18 µm aus. Die Einlehner Abrasion solcher gefällten Silikate liegt vorzugsweise im Bereich von weniger als 2,5 Verlust / 100 000 Umdrehungen, noch mehr bevorzugt im Bereich von 0,1 bis 2,3 mg Verlust / 100 000 Umdrehungen. Beispiele für gefällte Silikate mit verdickenden Eigenschaften sind ZEODENT®165, ZEODENT®167 und Sorbosil® TC15.

Gefällte Silikate mit vorteilhaften abrasiven Eigenschaften zeichnen sich vorzugsweise durch eine mittlere Teilchengröße in einem Bereich von 5 bis 12 µm. Die Einlehner-Abrasion solcher gefällten Silikate liegt vorzugsweise im Bereich von 2,5 bis 25 Verlust / 100 000 Umdrehungen. Beispiele für solche gefällten Silikate mit vorteilhaften abrasiven Eigenschaften sind ZEODENT®115, ZEODENT®117, Sorbosil® AC35, Sorbosil® AC39 und Sorbosil® AC77.

Ein Ziel des Einsatzes einer beliebigen Mischung von gefällten Silikaten ist es, einen mittleren bis hohen Abrasionsfaktor der Zahnpflege- und Zahnreinigungszusammensetzung und eine geeignete Verdickung der Zusammensetzung bereitzustellen. Der RDA-Wert der erfindungsgemäßen Zusammensetzungen liegt bevorzugt in einem Bereich von 30 bis 160, noch mehr bevorzugt in einem Bereich von 50 bis 130.

In einer bevorzugten Ausführungsform der vorliegenden Erfindung enthält die Mischung an gefällten Silikaten 4 bis 10 Teile, noch mehr bevorzugt 6 bis 8 Teile, an einem oder mehreren gefällten Silikaten mit vorteilhaften abrasiven Eigenschaften und 0,5 bis 2 Teile, noch mehr bevorzugt 0,7 bis 1,5 Teile, an einem oder mehreren gefällten Silikaten mit vorteilhaften verdickenden Eigenschaften.

Der vorstehend beschriebene Putzkörper ist vorzugsweise in eine thixotrope Matrix, die noch mehr bevorzugt stabil ist, eingebettet, damit er im unbewegt lagernden Produkt in Schwebe gehalten wird. Eine Matrix wird im Sinn der vorliegenden Offenbarung dann als stabil bezeichnet, wenn auch bei längerem Stehen keine Phasentrennung eintritt. Durch die thixotrope Eigenschaft der Matrix zeigt die erfindungsgemäße Zahnpflegezusammensetzung verbesserte rheologische Eigenschaften in dem vorstehend beschriebenen Sinn.

Im Ruhezustand ist die Zusammensetzung so viskos, dass ein Absinken des Putzkörpers und anderer Feststoffe wirksam verhindert wird, während die Zusammensetzung bei hoher mechanischer Belastung in ihrer scheinbaren Viskosität verringert wird und sie so problemlos aus der Spendervorrichtung austreten kann. Die verbesserten rheologischen Eigenschaften lassen sich beispielsweise anhand der scheinbaren Viskosität der Zusammensetzung als Funktion der Scherrate / mechanischen Belastung zeigen. Die Zusammensetzung zeigt eine höhere Viskosität bei einer niedrigen Scherrate / mechanischen Belastung und eine geringere Viskosität bei einer höheren Scherrate / mechanischen Belastung. Die niedrige und die hohe Scherrate / mechanische Belastung unterscheiden sich vorzugweise um mindestens einen Faktor 2, vorzugsweise um mindestens einen Faktor 4 bis vorzugsweise maximal einen Faktor 10. Die thixotrope Eigenschaft der erfindungsgemäßen Zahnpflegezusammensetzung kann beispielsweise durch deren Thixotropie-Index (TI) ausgedrückt werden, welcher das Verhältnis der scheinbaren Viskosität bei niedriger Scherrate /mechanischer Belastung dividiert durch die scheinbare Viskosität bei hoher Scherrate / mechanischer Belastung ist. Gemäß einer bevorzugten Ausführungsform liegt der Thixotropie-Index im Bereich von 1 bis 10, noch mehr bevorzugt im Bereich von 2 bis 10 und höchst bevorzugt im Bereich von 2,5 bis 8, jeweils bezogen auf einen Unterschied der Scherrate / mechanischen Belastung um einen Faktor im Bereich von 2 bis 10.

In jedem Fall weist die erfindungsgemäßen Zusammensetzung ohne Treibgas gemäß einer Ausführungsform der vorliegenden Erfindung sowohl im Zustand niedriger Scherrate / mechanischer Belastung als auch im Zustand einer hohen Scherrate / mechanischen Belastung eine dynamische Viskosität von 200 - weniger als 30.000 m Pa s, gemessen mit einem SVM™ 3001 Stabinger Viskosimeter™ von Anton Paar nach ASTM D7042 bei 20°C, 1013,25 mbar, auf. Vorzugsweise liegt die Viskosität der Zusammensetzung ohne Treibgas in einem Bereich von 400 - 10.000 m Pa s und noch mehr bevorzugt bei weniger als 10.000 m Pa s.

Insbesondere zur Bereitstellung dieser Matrix umfasst die erfindungsgemäße Zusammensetzung wenigstens ein Schichtsilikat. Das wenigstens eine Schichtsilikat kann grundsätzlich ein modifiziertes oder ein unmodifiziertes, natürlich vorkommendes oder künstliches Schichtsilikat oder ein Gemisch aus mehreren solcher Schichtsilikate sein. Für ein natürlich vorkommendes Schichtsilikat sei beispielhaft Hectorit genannt.

Das in der erfindungsgemäßen Zusammensetzung enthaltene wenigstens eine Schichtsilikat ist vorzugsweise ein künstliches Schichtsilikat. Schichtsilikate umfassen als Anion [Si₄O₁₀]⁴⁻-Ionen und als Kation Metallkationen, vorzugsweise Magnesium-, Natrium- und/oder Lithiumionen. Vorzugsweise sind in jeder Elementarzelle des Schichtsilikats SiO₄-Tetraeder jeweils in einer Ebene miteinander verkettet und bilden ein Schichtengitter. Die Metallkationen sind als Gegenionen vorhanden. In einer Elementarzelle sind vorzugsweise sechs oktaedrische Magnesiumionen zwischen zwei Schichten tetraedrischer Siliciumatome angeordnet. Diese Gruppen werden durch zwanzig Sauerstoffatome und vier Hydroxylgruppen ausgeglichen. Eine Vielzahl dieser Elementarzellen bildet dann einen scheibenförmigen Kristall.

Diese definierten scheibenförmigen Kristalle oder Mineralpartikel weisen vorzugsweise eine mittlere Dicke von 0,1 bis 5 nm, noch mehr bevorzugt eine mittlere Dicke von 0,2 bis 3 nm, und einen mittleren Durchmesser vorzugsweise im Bereich von 10 bis 50 nm, noch mehr bevorzugt im Bereich von 15 bis 35 nm auf. Die Größenverteilung der Partikel hat vorzugsweise eine Standardabweichung im Bereich von 5 bis 25 %, vorzugsweise im Bereich von 7,5 bis 20 %. Das Schichtsilikat umfasst oder besteht aus diesen Kristallen. Derartige künstliche Schichtsilikate sind im Handel beispielsweise unter dem Produktnamen Laponite bekannt und erhältlich.

Durch den Ersatz einiger Magnesium-Kationen durch einwertige Kationen, wie beispielsweise Lithium- oder Natrium-Kationen, bleiben in der Gitterstruktur der Elementarzelle, aufgrund der unterschiedlichen Wertigkeit dieser Ionen, einige für Magnesium vorgesehene Stellen unbesetzt. Dies führt zu einer negativen Gesamtladung, welche durch austauchbare Ionen, beispielsweise Natriumionen, kompensiert wird. Die einzelnen Schichten werden durch diese Ionen, beispielsweise die Natriumionen, die sich bevorzugt zwischen den Schichten, also außerhalb der regelmäßigen Gitterstruktur befinden, elektrostatisch verbunden.

In Dispersion in einem geeigneten Lösungsmittel, vorzugsweise in Wasser, liegt das Schichtsilikat in Form von den vorstehend beschriebenen scheibenförmigen Kristallen vor. Da sich beim Dispergieren eines solchen Schichtsilikats austauschbare Kationen herauslösen, verbleiben negativ geladene Schichten, die sich aufgrund ihrer Ladung abstoßen und gegeneinander verschiebbar sind. Die einzelnen scheibenförmigen Kristalle bilden so eine Gelstruktur aus, die jedoch bei Scherbeanspruchung unter Herabsetzung der Viskosität leicht zusammenbricht und sich danach im Ruhezustand schnell wieder aufbaut, so dass die thixotrope Eigenschaft entsteht.

Das wenigstens eine Schichtsilikat ist mit einem Anteil von 0,5 bis 5 Gew.-%, bezogen auf die Gesamtzusammensetzung, in der erfindungsgemäßen Zusammensetzung umfasst. Vorzugsweise beträgt der Anteil des wenigstens einen Schichtsilikats 1 bis 4,5 Gew.-%, noch mehr bevorzugt 2 bis 4 Gew.-%.

Die erfindungsgemäße Zahnpflege- und Zahnreinigungszusammensetzung umfasst weiterhin wenigstens ein pyrogenes Silikat. Gemäß einer Ausführungsform der vorliegenden Erfindung ist oder umfasst das wenigstens eine pyrogene Silikat ein hydrophiles pyrogenes Silikat. Gemäß einer weiteren Ausführungsform der vorliegenden Erfindung ist oder umfasst das wenigstens eine pyrogene Silikat ein hydrophobes pyrogenes Silikat. Ebenso umfasst sind gemäß einer weiteren erfindungsgemäßen Ausführungsform Mischungen von wenigstens einem hydrophilen pyrogenen Silikat und wenigsten einem hydrophoben pyrogenen Silikat.

Pyrogene Silikate werden, im Unterschied zu gefällten Silikaten nicht nasschemisch, sondern durch ein thermisches Verfahren, vorzugsweise durch eine Flammenhydrolyse, hergestellt und sie können ohne weitere Vermahlung oder Sprühtrocknung angewendet werden.

Die spezifische Oberfläche nach BET der erfindungsgemäß eingesetzten pyrogenen Silikate liegt vorzugsweise im Bereich von 10 bis 1000 m²/g, noch mehr bevorzugt im Bereich von 20 bis 800 m²/g, weiter bevorzugt im Bereich von 40 bis 600 m²/g und höchst bevorzugt im Bereich von 100 bis 300 m²/g. Die mittlere Primärteilchengröße, also die Größe der kleinsten Bauteile, die im pyrogenen Silikat mehr oder minder stark aggregiert und agglomeriert sind, liegt vorzugsweise im Bereich von 2 bis 70 nm, noch mehr bevorzugt im Bereich von 5 bis 50 nm.

Die direkt nach dem thermischen Verfahren erhaltenen pyrogenen Silikate sind aufgrund der frei zugänglichen Silanolgruppen (Si-OH) auf der Partikeloberfläche hydrophil und lassen sich somit mit Wasser benetzen. In einer Ausführungsform der vorliegenden Erfindung ist das /sind die in der erfindungsgemäßen Zusammensetzung enthaltene(n) pyrogene(n) Silikat(e) hydrophil. Hydrophile pyrogene Silikate sind im Handel erhältlich und können beispielsweise unter dem Namen Aerosil® 200 bezogen werden.

Erfindungsgemäß ist es aber in einer anderen Ausführungsform ebenfalls vorgesehen, dass das/die in der erfindungsgemäßen Zusammensetzung enthaltene(n) pyrogene(n) Silikat(e) oberflächenbehandelt sind und damit hydrophobe Eigenschaften aufweisen. Die Oberflächenbehandlung erfolgt vorzugsweise durch einen chemischen Nachbehandlungsschritt. Das so erzeugte hydrophobe Verhalten kommt durch die Reaktion von hydrophilen Silanolgruppen mit organischen Resten, vorzugsweise mit Silanen, beispielsweise Dichlordimethylsilan, Alkoxy-Silanen, Silazanen, Siloxanen, C₁-C₅-Kohlenstoffgruppen, beispielsweise CH₃-Gruppen, zustande. Hydrophobe pyrogene Silikate sind im Handel erhältlich und können beispielsweise unter dem Namen Aerosil R 812 S bezogen werden.

Das in Kombination mit dem wenigstens einen Schichtsilikat in der Zusammensetzung umfasste pyrogene, und vorzugsweise hydrophobe Silikat oder die Mischung aus zwei oder mehr pyrogenen Silikaten ist in der Zusammensetzung mit einem Anteil von 0,1 bis 2 Gew.-%, bezogen auf die Gesamtzusammensetzung, in der erfindungsgemäßen Zusammensetzung umfasst. Vorzugsweise beträgt der Anteil des wenigstens einen pyrogenen Silikats 0,15 bis 1,5 Gew.-%, noch mehr bevorzugt 0,2 bis 1,0 Gew.-%, jeweils bezogen auf die Gesamtzusammensetzung.

Gerade durch die Kombination des vorstehend beschriebenen wenigstens einen Schichtsilikats und des vorstehend beschriebenen wenigstens einen pyrogenen Silikats kann eine Matrix und eine Zusammensetzung bereitgestellt werden, die sich durch eine wesentliche und schnelle Rückkehr zu der Konsistenz im Ruhezustand auszeichnet, wenn die mechanische Belastung aufgehoben wird. Darüber hinaus hat sich überraschenderweise gezeigt, dass der Einsatz einer Silikat-Putzkörperkomponente mit verdickender Eigenschaft die vorteilhaften Eigenschaften der Matrix und der Zusammensetzung weiter positiv beeinflusst.

Die Grundrezeptur der erfindungsgemäßen Zahnpflegezusammensetzung zeichnet sich in einer bevorzugten Ausführungsform durch die Kombination von drei unterschiedlichen Silikat-Gruppen aus. Diese sind mindestens ein gefälltes Silikat als Putzkörper, mindestens ein pyrogenes Silikat und mindestens ein Schichtsilikat. Noch weiter bevorzugt wird bei dem Einsatz einer Mischung an gefällten Silikaten darauf geachtet, dass auch mindestens ein gefälltes Silikat mit verdickenden Eigenschaften, vorzugsweise mit einer mittleren Teilchengröße in einem Bereich von mehr als 12 bis 20 µm, zur Unterstützung der Konsistenz und der Matrix zugesetzt wird (vgl. Anspruch 10). Hierdurch können besonders vorteilhafte thixotrope Eigenschaften bereitgestellt werden.

Die erfindungsgemäße Zusammensetzung weist ferner wenigstens eine oberflächenaktive Substanz, nachfolgend auch als Tensid bezeichnet, auf. Pharmazeutisch und/oder kosmetisch akzeptable Tenside sind dem Fachmann grundsätzlich bekannt. Es können beispielsweise anionische, kationische, nichtionische oder amphotere Tenside hierfür verwendet werden. Gemäß einer Ausführungsform der vorliegenden Erfindung handelt es sich bei dem einen oder den mehreren Tensiden nicht um ein oder mehrere kationische Tenside. Beispielshaft seien als erfindungsgemäß einsetzbare Tenside 1-Hexadecanol, 1-Octadecanol, Polyacrylsäure, Polyethylenglykol, Cocamidopropyl Betaine, Natriumlaurylsulfat, Natriumlaurylsarkosinat, Trinatriumphosphat, Natriummethylcococyltaurat, Fettalkoholpolyglycoside, beispielsweise C₆-C₂₀-Alkylglykoside, einzeln oder in beliebigen Mischungen davon genannt.

In einer erfindungsgemäß bevorzugten Ausführungsform umfasst die Zusammensetzung als oberflächenaktive Substanz wenigstens ein nichtionisches Tensid. Noch mehr bevorzugt umfasst die erfindungsgemäße Zusammensetzung nur ein oder mehrere nichtionische Tenside und ist somit im Wesentlichen frei von Tensiden ausgewählt aus der Gruppe, bestehend aus anionischen, kationischen und amphoteren Tensiden. Der Ausdruck "im Wesentlichen frei von Tensiden" bedeutet im Sinne der vorliegenden Erfindung, dass der Anteil an einem oder mehrerer dieser Tenside, außer der nichtionischen Tenside, bezogen auf die Gesamtzusammensetzung, weniger als 0,5 Gew.-%, vorzugsweise weniger als 0,3 Gew.-%, noch mehr bevorzugt weniger als 0,1 Gew.-% und höchst bevorzugt 0 Gew.-%, jeweils bezogen auf die Gesamtzusammensetzung, beträgt. Etwaige nicht vermeidbare Verunreinigungen sollen hierbei außer Acht bleiben.

Das nichtionische Tensid ist vorzugsweise ein C₆-C₂₀-Alkylglykosid, vorzugsweise ein C₈-C₁₆-Alkylglykosid. Derartige Alkylglykoside sind im Handel beispielsweise unter dem Markennamen Plantacare oder dem INCI-Namen Coco-Glucoside erhältlich.

Das wenigstens eine nichtionisches Tensid, vorzugsweise das C₆-C₂₀-Alkylglykosid, noch mehr bevorzugt das C₈-C₁₆-Alkylglykosid, kann gemäß einer weiteren Ausführungsform auch in Kombination mit einem oder mehreren anderen anionischen, kationischen oder amphoteren Tensiden vorliegen. Umfasst ist beispielsweise eine Kombination mit Natriumlaurylsulfat und/oder Natriumlaurylsarkosinat oder mit Cocamidopropyl Betaine oder mit einem Gemisch aus Natriumlaurylsulfat und Cocamidopropyl Betaine.

Der oder den oberflächenaktiven Substanzen kommt in der erfindungsgemäßen Zusammensetzung sowohl eine Funktion bei der Bildung von Schaum als auch bei der Regelung der Viskosität der Zusammensetzung zu.

Der Anteil der wenigstens einen oberflächenaktiven Substanz in der erfindungsgemäßen Zusammensetzung beträgt 0,2 bis 5,0 Gew.-%, bezogen auf die Gesamtzusammensetzung. Vorzugweise beträgt der Anteil 0,3 bis 2,5 Gew.-%, wiederum bezogen auf die Gesamtzusammensetzung. Enthält die erfindungsgemäße Zusammensetzung als oberflächenaktive Substanz nur ein oder mehrere nichtionische Tenside, so ist dessen oder deren Anteil in der erfindungsgemäßen Zusammensetzung 0,2 bis 5,0 Gew.-%, vorzugsweise 0,3 bis 2,5 Gew.-%, bezogen auf die Gesamtzusammensetzung.

Die erfindungsgemäße Zusammensetzung umfasst weiterhin wenigstens ein Lösungsmittel. Das Lösungsmittel ist vorzugsweise Wasser oder umfasst vorzugsweise Wasser in einem Lösungsmittelgemisch. Noch mehr bevorzugt ist das Wasser für kosmetische oder pharmazeutische Formulierungen geeignet. Weitere pharmazeutisch und/oder kosmetisch akzeptable Lösungsmittel, die einzeln oder in Lösungsmittelgemischen, vorzugsweise in Kombination mit Wasser, eingesetzt werden können, sind dem Fachmann bekannt.

Der Anteil des wenigstens einen Lösungsmittels ist der Rest auf 100 Gew.-% (ad. 100 Gew.-%), bezogen auf die Gesamtzusammensetzung. Das heißt die Gew.-%-Anteile der wesentlichen und gegebenenfalls vorhandenen optionalen Bestandteile der Zusammensetzung sind immer so gewählt, dass sich in der Summe 100 Gew.-% ergeben.

Die erfindungsgemäße Zusammensetzung ist zur Reinigung und Pflege der Zähne geeignet. Im Besonderen ist die erfindungsgemäße Zusammensetzung zur Anwendung bei der Zahnreinigung und -pflege mittels einer Zahnputzvorrichtung für die gleichzeitige Reinigung mehrerer, vorzugsweise aller Zähne eines Benutzers, geeignet. Hierbei wird die erfindungsgemäße Zusammensetzung durch Aufbringen der Zusammensetzung auf einen Mundeinsatz oder mehrere Mundeinsätze der Zahnputzvorrichtung dargereicht. Ebenso ist die erfindungsgemäße Zusammensetzung zur Prävention von Zahn- und Zahnfleischerkrankungen geeignet.

Das thixotrope Verhalten der erfindungsgemäßen Zusammensetzung ermöglicht es, die Putzkörper stabil in Schwebe zu halten und so eine gleichmäßige und sparsame Verteilung der Zusammensetzung auf allen Reinigungsflächen des Mundeinsatzes oder der Mundeinsätze zu gewährleisten. Es kommt dabei weder zu einer Überdosierung an Putzkörpern, was zu einer mechanischen Schädigung des Zahnschmelzes und zu einem unerwünschten Abrieb der Zähne führen könnte, noch kommt es zu einer Unterdosierung an Putzkörpern, was zu einer zu geringen Reinigung der Zähne führen könnte. Außerdem schäumt die erfindungsgemäße Zusammensetzung nicht zu stark.

Durch die Kombination der vorstehend genannten wesentlichen Bestandteile wird eine Zusammensetzung ermöglicht, die besonders zur gleichmäßigen, sparsamen und einfachen Applikation auf den oder die Mundeinsätze der Zahnputzvorrichtung geeignet ist. Durch die Kombination der vorstehend genannten wesentlichen Bestandteile kann die gewünschte Matrix stabil eingestellt und die Putzkörper während der Lagerung der Zusammensetzung in Schwebe gehalten werden. Darüber hinaus kann eine stabile Schaumbildung bei der Applikation erreicht werden.

Die erfindungsgemäße Zusammensetzung ist zur Reinigung und Pflege der Zähne, der Zahnhälse und des Zahnfleisches und insbesondere zur Vorbeugung und/oder Behandlung von Zahn- und Zahnfleischerkrankungen, insbesondere von Karies, Parodontose und Plaque, geeignet. Sie wird gemäß einer Ausführungsform der vorliegenden Erfindung hierfür verwendet.

Der erfindungsgemäßen Zahnpflegezusammensetzung kann gemäß einer Ausführungsform wenigstens ein Feuchthaltemittel zugesetzt sein. Feuchthaltemittel dienen zum einen dazu, ein Austrocknen der Zusammensetzung zu verhindern. Das oder die Feuchthaltemittel können aber auch als Konsistenzgeber und als Kältestabilisator fungieren. Feuchthaltemittel können ferner auch zur Suspensionsvermittlung und zur Geschmacks-und/oder Glanzbeeinflussung dienen. Pharmazeutisch und/oder kosmetisch akzeptable Feuchthaltemittel sind dem Fachmann bekannt. Beispielhaft seien Glycerin, Sorbitol, hydrogeniertes Stärkehydrolysat, 1,2-Propylenglycol und weitere mehrwertige Alkohole mit mindestens 2 OH-Gruppen, wie Mannitol und Xylitol, oder beliebige Mischungen von diesen Feuchthaltemitteln genannt.

Das Feuchthaltemittel oder das Gemisch aus Feuchthaltemitteln ist in der erfindungsgemäßen Zusammensetzung vorzugsweise mit einem Anteil von weniger als 20 Gew.-%, noch mehr bevorzugt von weniger als 15 Gew.-% enthalten. Der Anteil an dem oder der Feuchthaltemittel liegt vorzugweise im Bereich von 1 bis 14 Gew.-%, noch mehr bevorzugt im Bereich von 2 bis 12 Gew.-%.

Besonders bevorzugt ist der Einsatz von Xylitol als Feuchthaltemittel. Noch mehr bevorzugt umfasst die erfindungsgemäße Zahnpflegezusammensetzung außer Xylitol keine anderen mehrwertigen Alkohole und/oder andere Feuchthaltemittel. So ist die erfindungsgemäße Zusammensetzung gemäß eine bevorzugten Ausführungsform insbesondere im Wesentlichen frei von Sorbitol und/oder Glycerin und/oder 1,2-Propylenglycol. Der Ausdruck "im Wesentlichen frei von Sorbitol und/oder Glycerin und/oder 1,2-Propylenglycol" bedeutet im Sinne der vorliegenden Erfindung, dass der Anteil an einem oder mehrerer dieser Feuchthaltemittel, außer Xylitol, bezogen auf die Gesamtzusammensetzung, weniger als 5 Gew.-%, vorzugsweise weniger als 3 Gew.-%, noch mehr bevorzugt weniger als 1 Gew.-% und höchst bevorzugt 0 Gew.-%, jeweils bezogen auf die Gesamtzusammensetzung, beträgt. Etwaige nicht vermeidbare Verunreinigungen sollen hierbei außer Acht bleiben. Der Anteil an Xylitol, bezogen auf die Gesamtzusammensetzung, beträgt vorzugsweise weniger als 20 Gew.-%, noch mehr bevorzugt von weniger als 15 Gew.-%, noch weiter bevorzugt 1 bis 14 Gew.-% und höchst bevorzugt 2 bis 12 Gew.-%.

Optional können die erfindungsgemäßen Zusammensetzungen ferner wenigstens einen Viskositätsverstärker umfassen. Gemäß einer Ausführungsform ist der Viskositätsverstärker ausgewählt aus der Gruppe, bestehend aus Polysacchariden, wie Cellulosen, Xanthan Gum, Carrageenane, etc., und Polymeren, wie Acrylate, Polyacrylate, insbesondere vernetzte Polyacrylate, C10-30-Alkyl-Acrylat-Crosspolymer, anderer verdickender Polymere und beliebigen Mischungen davon. Viskositätsverstärkende Cellulosen umfassen sowohl nicht-derivatisierte, wasserunlösliche Cellulosen, also auch derivatisierte Cellulosen, wie Carboxymethylcellulose und Hydroxyethylcellulose. Neben den Acrylaten und Polyacrylaten können beispielsweise Polyvinylalkohol und/oder Polyvinylpyrrolidon als viskositätsverstärkende Polymere eingesetzt werden.

Der Anteil des wenigstens einen Viskositätsverstärkers in der erfindungsgemäßen Zusammensetzung beträgt vorzugsweise 0 bis 5 Gew.-%, bezogen auf die Gesamtzusammensetzung, noch mehr bevorzugt beträgt der Anteil des wenigstens einen Viskositätsverstärkers, sofern in der Zusammensetzung vorhanden, 0,01 bis 4 Gew.-%, bezogen auf die Gesamtzusammensetzung.

Erfindungsgemäß bevorzugte Viskositätsverstärker sind Xanthan Gum und Polyacrylate, insbesondere vernetzte Polyacrylate. Diese können einzeln oder in einer beliebigen Mischung vorliegen. Im Fall einer Mischung, ist eine Mischung von Xanthan Gum und Polyacrylat besonders bevorzugt. Das Gewichtsverhältnis in der Mischung von Xanthan Gum und Polyacrylat, insbesondere vernetztem Polyacrylat, liegt vorzugsweise in einem Bereich von 1-3 Teilen Xanthan Gum zu 3-1 Teilen Polyacrylat, insbesondere vernetztem Polyacrylat. Noch mehr bevorzugt liegt das Gewichtsverhältnis von Xanthan Gum zu Polyacrylat, insbesondere vernetztem Polyacrylat, im Bereich von 1-2 Teilen zu 2-1 Teilen. Höchst bevorzugt ist das Gewichtsverhältnis in der Mischung 1 Teil Xanthan Gum zu 1 Teil Polyacrylat, insbesondere vernetztem Polyacrylat.

Gemäß einer bevorzugten Ausführung ist die erfindungsgemäße Zusammensetzung jedoch im Wesentlichen frei von Cellulosen, insbesondere von Carboxymethylcellulose und/oder Hydroxyethylcellulose und/oder anderen Polysacchariden, insbesondere von Xanthan, Xanthan Gum, Alginate und/oder Carragene. Gemäß einer weiteren bevorzugten Ausführungsform ist die erfindungsgemäße Zusammensetzung im Wesentlichen frei von Polymeren, insbesondere von Acrylaten, Polyacrylaten, insbesondere vernetzten Polyacrylaten, C10-30-Alkyl-Acrylat-Crosspolymeren, Polyvinylalkohol und /oder Polyvinylpyrrolidon, die sich als Viskositätsverstärker eignen. Gemäß einer noch weiteren bevorzugten Ausführungsform ist die erfindungsgemäße Zusammensetzung im Wesentlichen frei von den vorstehend genannten Polysacchariden und Polymeren mit viskositätsverstärkenden Eigenschaften. Der Ausdruck "im Wesentlichen frei" bedeutet hier im Sinne der vorliegenden Erfindung, dass der Anteil an einem oder mehrerer dieser Viskositätsverstärker, bezogen auf die Gesamtzusammensetzung, weniger als 1 Gew.-%, vorzugsweise weniger als 0,5 Gew.-%, noch mehr bevorzugt weniger als 0,25 Gew.-% und höchst bevorzugt 0 Gew.-%, jeweils bezogen auf die Gesamtzusammensetzung, beträgt. Etwaige nicht vermeidbare Verunreinigungen sollen hierbei außer Acht bleiben.

Weiterhin ist es erfindungsgemäß bevorzugt, dass die Zahnpflege- und Zahnreinigungszusammensetzung wenigstens einen Wirk- oder Inhaltsstoff umfasst. Der mindestens eine Wirkstoff ist beispielsweise ausgewählt aus der Gruppe, bestehend aus Hydroxylapatit, beispielsweise synthetischem Nano-Hydroxylapatit, oder weiteren remineralisierenden oder keramisierenden Stoffen, einem Fluoridwirkstoff, einem Mittel zur Zahnfleischpflege, einem desinfizierenden Bestandteil, einem Mittel zur Reduktion von Zahnbelägen, einem entzündungshemmenden Mittel und einer beliebigen Mischung davon.

Der Anteil des wenigstens einen oder der weiteren Wirkstoffe in der erfindungsgemäßen Zusammensetzung beträgt 0 bis 10 Gew.-%, bezogen auf die Gesamtzusammensetzung. Vorzugweise beträgt der Anteil des wenigstens einen oder der Wirkstoffe 0,1 bis 5,0 Gew.-%, noch mehr bevorzugt 1,0 bis 4,0 Gew.-%, jeweils bezogen auf die Gesamtzusammensetzung.

Als Fluorid-Wirkstoff wird besonders bevorzugt ein Aminfluorid, Natriumfluorid oder Zinnfluorid oder eine Mischung davon verwendet. Der oder die Fluorid-Wirkstoffe sind, sofern vorhanden, vorzugsweise mit einem Anteil von 0,005 bis 5 Gew.-%, bezüglich des Fluorids und bezogen auf die Gesamtzusammensetzung enthalten. Noch mehr bevorzugt beträgt der Anteil 0,01 bis 2,5 Gew.-%, höchst bevorzugt liegt der Anteil im Bereich von 0,02 bis 1 Gew.-%.

Alternativ zum Fluorid-Wirkstoff kann die erfindungsgemäße Zusammensetzung Hydroxylapatit enthalten. In einer möglichen erfindungsgemäßen Ausführungsform enthält die Zahnpflegezusammensetzung entweder Fluoride, als weiteren Wirkstoff, oder Hydroxylapatit oder weitere remineralisierende Stoffe und Kombinationen aus Hydroxylapatit und einem oder mehreren weiteren remineralisierenden Stoffen.

Der Anteil an Hydroxylapatit oder weiterer remineralisierender Stoffe und Kombinationen aus Hydroxylapatit und einem oder mehreren weiteren remineralisierenden Stoffen beträgt vorzugsweise 1 bis 30 Gew.-%, noch mehr bevorzugt 1 bis 15 Gew.-%, jeweils bezogen auf die Gesamtzusammensetzung.

Als Mittel zur Zahnfleischpflege wird besonders bevorzugt Allantoin, Panthenol, Extrakte aus Arnika, Myrrhe, Ratanhia, Salbei, Echinacea, Kamille, Rosmarinöl, Thymianöl oder Aloe oder eine beliebige Mischung von diesen Substanzen verwendet. Das Mittel zur Zahnfleischpflege ist, falls vorhanden, vorzugsweise mit einem Anteil von 0,1 bis 3 Gew.-%, noch mehr bevorzugt mit einem Anteil von 0,15 bis 2 Gew.-%, bezogen auf die Gesamtzusammensetzung enthalten.

Als Mittel zur Reduktion von Zahnbelägen kann beispielsweise Chlorhexidindigluconat, Zinkaspartat, Arginin, Phosphate, beispielsweise Natriumtripolyphosphat, Cetylpyridiniumchlorid oder eine beliebige Mischung von diesen verwendet werden. Das Mittel zur Reduktion von Zahnbelägen ist, falls vorhanden, vorzugsweise mit einem Anteil von 0,2 bis 3 Gew.-%, noch mehr bevorzugt mit einem Anteil von 0,5 bis 2 Gew.-%, bezogen auf die Gesamtzusammensetzung enthalten.

Weitere Wirkstoffe umfassen auch entzündungshemmende Mittel, wie Aloevera, auch in Form von Extrakten oder in Form von Konzentraten, und desinfizierende Bestandteile, wie Bakterizide, Silberionen, Mikrosilber, Nanosilber, Zinksalze, beispielsweise Zinkcitrat und/oder Zinkchlorid. Sie sind, falls vorhanden, vorzugsweise mit einem Anteil von 0,05 bis 3 Gew.-%, noch mehr bevorzugt mit einem Anteil von 0,1 bis 2 Gew.-%, weiter bevorzugt mit einem Anteil von 0,2 bis 1 Gew.-%, jeweils bezogen auf die Gesamtzusammensetzung, enthalten.

Es ist weiterhin erfindungsgemäß bevorzugt, dass die bereitgestellte Zusammensetzung übliche Hilfs- und Zusatzstoffe ausgewählt aus der Gruppe, bestehend aus Aromastoffe, Süßungsmittel, Konservierungsmittel, Pigmente, Farbstoffe, Puffersubstanzen und einer beliebigen Mischung davon umfasst.

So kann die erfindungsgemäße Zusammensetzung beispielsweise ein oder mehrere Farbpigmente umfassen. Pharmazeutisch und/oder kosmetisch akzeptable Farbpigmente sind dem Fachmann bekannt. Diese können einzeln oder in beliebigen Mischungen davon eingesetzt werden. Beispielsweise wird als Farbpigment Puricolor Blue PBL (15:3), gegebenenfalls auch in Mischung mit weiteren Farbpigmenten eingesetzt. Der Anteil an Farbpigment, sei es ein Farbpigment oder eine Mischung aus mehreren Farbpigmenten, beträgt vorzugsweise 0 bis 1,0 Gew.-%, vorzugsweise 0,2 bis 1,0 Gew.-%, bezogen auf die Gesamtzusammensetzung. Noch mehr bevorzugt beträgt der Bereich 0,3 bis 0,8 Gew.-%.

Die erfindungsgemäße Zusammensetzung kann ferner einen oder mehrere Aromastoffe umfassen. Besonders bevorzugt umfasst der Aromastoff Anethol, Menthol, grüne Minze ("Spearmint"), Eucalypthus oder Limonen oder eine beliebige Kombination von diesen bevorzugten Aromastoffen. Der Anteil an Aromastoffen, sei es ein Aromastoff oder eine Mischung aus mehreren Aromastoffen, beträgt vorzugsweise 0 bis 1,5 Gew.-%, vorzugsweise 0,1 bis 1,3 Gew.-%, bezogen auf die Gesamtzusammensetzung. Noch mehr bevorzugt beträgt der Anteil 0,15 bis 1,1 Gew.-%.

Als Konservierungsmittel wird in einer erfindungsgemäßen Ausführungsform vorzugsweise Natriummethylparaben, Natriumbenzoat, Kaliumsorbat oder eine beliebige Mischung davon, vorzugsweise mit einem Anteil in einem Bereich von 0,05 bis 2 Gew.-%, noch mehr bevorzugt mit einem Anteil in einem Bereich von 0,1 bis 1 Gew.-%, jeweils bezogen auf die Gesamtzusammensetzung, eingesetzt.

Als weitere optionale Bestandteile kann die erfindungsgemäße Zusammensetzung umfassen wenigstens ein Bindemittel, wenigstens einen Lösungsvermittler, wenigstens ein Netzmittel, wenigstens einen Süßstoff, wenigstens ein Bleichmittel, einen Pflanzenextrakt und/oder wenigstens einen pH-Regler. Die erfindungsgemäße Zusammensetzung kann als optionalen Bestandteil auch einen oder mehrere weitere Stabilisatoren, wie beispielsweise Natriumgluconat (INCI: Sodium Gluconate) umfassen. Dieser wenigstens eine weitere Stabilisator kann eine Entmischung der Emulsion verhindern und/oder eine Zersetzung der anderen Bestandteile der Zusammensetzung, beispielsweise der Wirkstoffe, verhindern. Natriumgluconat verhindert beispielsweise nicht nur eine Entmischung der erfindungsgemäßen Zusammensetzung, es verhindert darüber hinaus auch eine oxidative Zersetzung der weiteren Bestandteile, insbesondere der Wirkstoffe, der Zusammensetzung. Diese optionalen Bestandteile können einzeln oder in beliebigen Kombinationen miteinander in der erfindungsgemäßen Zusammensetzung umfasst sein.

Die Wirkstoffe und Hilfs- oder Zusatzstoffe der Zusammensetzung zur Mund- und/oder Zahnreinigung und -pflege können grundsätzlich die sein, wie sich auch für bereits bekannte Zahnreinigungsmittel, wie etwa Zahncremes, Zahnreinigungsgelen und dergleichen, eingesetzt werden. Dennoch haben sich bestimmte Inhalts- und Hilfs- oder Zusatzstoffe und/oder deren Kombination miteinander als besonders vorteilhaft herausgestellt, um eine Zusammensetzung bereitzustellen, die gleichmäßig, sparsam und einfach auf den Mundeinsatz oder die Mundeinsätze der Zahnputzvorrichtung zum gleichzeitigen Reinigen von mehrerer, vorzugsweise aller Zähne eines Benutzers, appliziert werden kann. Darüber hinaus stellt die Zusammensetzung sicher, dass genügend Wirkstoff appliziert wird, aber gleichzeitig eine Überdosierung vermieden wird.

Alle Bestandteile der Zusammensetzung sind in einem Reinheitsgrad, der für Zahnpflegezusammensetzungen geeignet und notwendig ist. Werden für bestimmte Hilfs- oder Zusatzstoffe hierin keine Angaben zu deren Gew.-%-Anteil gemacht, bedeutet dies, dass der Fachmann die bevorzugten Bereiche kennt oder einfach ermitteln kann.

Der pH-Wert der erfindungsgemäßen Zahnpflege- und Zahnreinigungszusammensetzung liegt vorzugsweise im Bereich von 4,0 bis 8,0, noch mehr bevorzugt im Bereich von 4,5 bis 7,5.

Ebenfalls im Umfang der vorliegenden Erfindung mit umfasst sind solche Zusammensetzungen, die, mit Ausnahme von üblichen Verunreinigungen, nur aus den vorstehend genannten wesentlichen, und gegebenenfalls einem oder mehreren der optionalen Bestandteile, bestehen.

Die Angabe Gew.-%, wie in der vorliegenden Patentanmeldung verwendet, bedeutet, sofern nichts anderes angegeben wird, dass der Anteil auf das Gesamtgewicht der Zusammensetzung ohne Treibmittel bezogen ist.

Eine besonders bevorzugte erfindungsgemäße Zusammensetzung umfasst:

| | |
|---|---|
| Putzkörper: | 5 bis 15 Gew.-% |
| Pyrogenes Silikat: | 0,15 bis 1 Gew.-% |
| Schichtsilikat, vorzugsweise Laponite: | 1 bis 4 Gew.-% |
| Propylenglycol: | 0 bis 2 Gew.-% |
| Xylitol: | 4 bis 12 Gew.-% |
| Olaflur: | 0,4 bis 0,8 Gew.-% |
| d-Panthenol: | 0,4 bis 0,8 Gew.-% |
| nichtionisches Tensid: | 0,3 bis 0,7 Gew.-% |
| Konservierungsmittel: | 0,1 bis 0,2 Gew.-% |
| Xanthan Gum: | 0 bis 0,07 Gew.-% |
| Acrylate/C10-30-Alkyl-Acrylat-Crosspolymer: | 0 bis 0,07 Gew.-% |
| Aroma: | 0,1 bis 0,3 Gew.-% |

sowie ein Lösungsmittel, vorzugsweise Wasser ad 100 Gew.-%. In dieser bevorzugten Zusammensetzung umfasst der Putzkörper mindestens ein gefälltes Silikat gemäß der vorstehend beschriebenen Spezifikation. Noch mehr bevorzugt umfasst der Putzkörper mindestens ein gefälltes Silikat mit einer mittleren Teilchengröße in einem Bereich von 5 bis 12 µm und mindestens ein gefälltes Silikat mit einer mittleren Teilchengröße in einem Bereich von mehr als 12 bis 20 µm. Das mindestens eine gefällte Silikat mit vorteilhaften abrasiven Eigenschaften ist vorzugsweise mit 5 bis 10 Teilen, bezogen auf einen Teil des gefällten Silikats mit vorteilhaften verdickenden Eigenschaften, innerhalb der angegebenen Gew.-% enthalten.

In einer Ausführungsform der vorliegenden Erfindung besteht die Basisformulierung der Zusammensetzung aus den im vorgehenden Abschnitt genannten Komponenten mit den angegebenen Anteilen. Dieser Basisformulierung können zur Bereitstellung der fertigen Zusammensetzung ohne Treibgas ergänzend zu diesen Komponenten wenigstens ein Wirkstoff, beispielsweise ausgewählt aus der Gruppe, bestehend aus Hydroxylapatit oder weiteren remineralisierenden oder keramisierenden Stoffen, einem Fluoridwirkstoff, einem Mittel zur Zahnfleischpflege, einem desinfizierenden Bestandteil, einem Mittel zur Reduktion von Zahnbelägen, einem entzündungshemmenden Mittel, einem Konservierungsmittel und einer beliebigen Mischung davon, und/oder wenigstens ein üblicher Hilfs- und Zusatzstoffe ausgewählt aus der Gruppe, bestehend aus Aromastoffe, Süßungsmittel, Konservierungsmittel, Pigmente, Farbstoffe, Puffersubstanzen und einer beliebigen Mischung davon, in den vorstehend spezifizierten Anteilen, sowie ein Mittel zum Einstellen des pH-Wertes zugesetzt werden.

Diese besonders bevorzugte Zusammensetzung kann entweder, abgesehen von üblichen Verunreinigungen, nur aus diesen Bestandteilen bestehen, oder sie kann darüber hinaus noch 0,3 bis 1,0 Gew.-% Chlorophyllin und/oder 0,5 bis 1,5 Gew% Anethol oder Menthol oder Limonen oder eine beliebige Mischung davon umfassen. Darüber hinaus können in der Zusammensetzung noch umfasst sein Saccharin oder ein anderer Süßstoff, ein pH-Regler, PEG-40 hydriertes Rizinusöl (INCI: PEG-40 Hydrogenated Castor Oil) und/oder Natriumgluconat (INCI: Sodium Gluconate). Gemäß einer weiteren erfindungsgemäßen Ausführungsform besteht die Zusammensetzung, abgesehen von üblichen Verunreinigungen, nur aus den genannten wesentlichen und einem oder mehrerer der genannten optionalen Bestandteile.

Geeignete Verfahren zum Herstellen der erfindungsgemäßen Zusammensetzungen sind dem Fachmann bekannt.

Bei der Verwendung der erfindungsgemäßen Zusammensetzung ist es erfindungsgemäß vorzugsweise vorgesehen, dass die erfindungsgemäße Zusammensetzung ausgehend von der thixotropen Mischung in einem zweiten Stadium in einen Schaum, vorzugsweise in ein Schaumspray, mit einer bestimmten Lebensdauer oder in ein Aerosol überführt wird. Deshalb kommt den oberflächenaktiven Substanzen und/oder den Viskositätsverstärkern in der Zusammensetzung eine besondere Bedeutung zu. Bezüglich besonders bevorzugter Substanzen und Kombinationen davon wird auf die vorstehende Offenbarung verwiesen, in der diese Substanzen im Einzelnen diskutiert werden.

Gemäß einem weiteren Aspekt betrifft die vorliegende Erfindung also die vorstehend im Detail beschriebene Zahnpflege- und Zahnreinigungszusammensetzung in Kombination mit einem in der Zusammensetzung enthaltenen Treibgas oder einem Treibgasgemisch. Vorzugsweise ist das Treibgas oder Treibgasgemisch in der erfindungsgemäßen Zusammensetzung in einer Menge von wenigstens 0,2 Gew.-%, noch mehr bevorzugt von wenigstens 0,5 Gew.-%, noch mehr bevorzugt von wenigstens 1 Gew.-% enthalten. Weiterhin ist es bevorzugt, dass der Gehalt an Treibgas oder Treibgasgemisch in der Zusammensetzung nicht höher als 15 Gew.-% und noch mehr bevorzugt nicht höher als 13 Gew.-% ist, da das Mittel ansonsten bei der Applikation und Verwendung einen zu voluminösen Schaum bilden würde. Das Treibgas oder Treibgasgemisch fungiert also als Schaumbildner. Am bevorzugtesten ist es, dass der Gehalt an Treibmittel oder Treibmittelgemisch in der Zusammensetzung in einem Bereich von 2,5 bis 12 Gew.-% liegt.

Die vorliegende Erfindung betrifft somit in einem weiteren Aspekt auch ein Aerosol oder einen Schaum, das/der aus der erfindungsgemäßen Zusammensetzung gebildet wird und das/der gleichmäßig auf den oder die Mundeinsätze verteilt werden kann. Das Aerosol oder der gebildete Schaum ist für einen ausreichenden Zeitraum stabil und haftet damit für einen ausreichenden Zeitraum an den Mundeinsätzen. Ein Abfließen der Zusammensetzung bzw. des daraus gebildeten Schaums oder der Schaumtropfen oder der Aerosoltropfen vor dem Einsetzen der Mundeinsätze in den Mund kann somit vermieden werden.

Aerosole und Schäume bieten den Vorteil einer langen Haltbarkeit, einer exakten Dosierung und einer sparsamen Anwendung. Darüber hinaus können in den Spender keine Mikroorganismen eindringen, so dass auch nicht konservierte Produkte möglich sind.

Ein Aerosol ist ein heterogenes Gemisch nicht gasförmiger Stoffe in einem Gas. Es entsteht erst durch Betätigung des Auslassventils einer geeigneten Spender- und Speichervorrichtung für die erfindungsgemäße Zahnreinigungs- und -pflegezusammensetzung und wird damit erst bei Bedarf und in der benötigten Menge hergestellt. Der Schaum ist das finale Produkt, welches durch die Spender- und Speichervorrichtung aus der erfindungsgemäßen Zusammensetzung gebildet wird. Ein Schaum umfasst gasförmige Bläschen, die von festen oder flüssigen Wänden eingeschlossen sind. Die in der Spendervorrichtung enthaltene Zusammensetzung sollte Tenside oder ähnliche Vernetzungsmittel, sowie vorzugsweise Schaumstabilisatoren enthalten, die eine ausreichend stabile Schaumerzeugung ermöglichen. In Abhängigkeit der speziellen Eigenschaften der in der Zusammensetzung enthaltenen oberflächenaktiven Stoffe entstehen Schaumbläschen mit unterschiedlicher Größe, Wandstärke und Lebensdauer. Grundsätzlich ist die Lebensdauer von flüssigen Schäumen begrenzt. Diese kann von der Viskosität der den Schaum bildenden Flüssigkeit, also der hierin beschriebenen Zusammensetzung, und/oder den in der Zusammensetzung enthaltenen Stoffen abhängen.

Gemäß einem weiteren Aspekt betrifft die vorliegende Erfindung ein System, welches die hierin beschriebene erfindungsgemäße Zahnpflege- und Zahnreinigungszusammensetzung, und einen Behälter bzw. Spender mit Mitteln zur Bevorratung und Abgabe der Zahnpflege- und Zahnreinigungszusammensetzung umfasst. Die Zahnpflege- und Zahnreinigungszusammensetzung kann mit einem Treibgas oder Treibgasgemisch versetzt sein.

Damit solche Systeme ein geeignetes Aerosol bzw. einen geeigneten Schaum produzieren und abgeben können, spielen neben der thixotropen Zusammensetzung noch andere technische Parameter wie beispielsweise das Verhältnis von Wirkstofflösung zu Treibgas, die Art des Treibgases, die Größe der Ventilöffnung und/oder die Größe der Sprühkopföffnung eine Rolle. Die Größe der Ventilöffnung und die Größe der Sprühkopföffnung stehen in direkter Korrelation zur Tröpfchengröße des Aerosols oder des Schaums. Für den Ventilkopf liegt sie vorzugsweise zwischen 0,2 mm und 4 mm, für den Sprühkopf vorzugsweise zwischen 0,4 mm und 6 mm.

Erfindungsgemäß umfasst der Spender ein Mittel zur Bevorratung der erfindungsgemäßen Zusammensetzung, ein Fördersystem zum Befördern der Zusammensetzung und ein Auslassventil zum Abgeben der Zusammensetzung. Das Fördersystem befördert die Zusammensetzung vorzugsweise mittels eines Treibgases, Druckluft oder über ein Pumpsystem nach außen. Der Spender ist dabei ausreichend druckstabil gegenüber beispielsweise dem Expansionsdruck der treibgashaltigen Zusammensetzung oder des Treibgases ausgebildet. Die Zusammensetzung und der Spender wirken derart zusammen, dass bei Betätigung des Auslassventils das Zahnreinigungsmittel, vorzugsweise das treibgashaltige Zahnreinigungsmittel, aus dem Auslassventil austritt und zeitlich verzögert am Applikationsort weiter aufschäumt bzw. expandiert. Das Zahnreinigungsmittel wird vorzugsweise in einer strangförmigen Form abgegeben und ist zum Zeitpunkt des Austritts vorzugsweise nur wenig aufgeschäumt. Wenig aufgeschäumt bedeutet in diesem Zusammenhang, dass die Zusammensetzung zu diesem Zeitpunkt vorzugsweise höchstens 20 %, noch mehr bevorzugt höchstens 10 % ihres endgültigen Volumens aufweist.

In einer erfindungsgemäßen Ausführungsform befördert das Fördersystem die Zusammensetzung mittels eines Treibgases oder Treibgasgemisches, Druckluft oder über ein Pumpsystem aus dem Spender. Die Austrittsöffnung des Spenders, beispielsweise die Schaumdüse, ist vorzugsweise derart ausgestaltet, dass eine gleichmäßige, zielgenaue und sparsame Verteilung der erfindungsgemäßen Zusammensetzung auf dem oder den Mundeinsätzen gewährleistet wird. Das Auslassventil ist vorzugsweise manuell betätigbar.

Gemäß einer bevorzugten Ausführungsform ist der Spender eine Aerosoldose bzw. Spraydose. Hierbei handelt es sich vorzugsweise um einen metallischen Behälter oder vorzugsweise um einen Kunststoffbehälter, der die erfindungsgemäße Zusammensetzung enthält und aufgrund des Einsatzes eines Treibgases oder Treibgasgemisches unter Druck steht. Durch den Innendruck der Spraydose wird der Inhalt genau dann freigesetzt, wenn man das Auslassventil betätigt. Ein Teil des Treibgases oder Treibgasgemisches liegt gelöst in der Wirkstofflösung, also der eigentlichen Zusammensetzung der vorliegenden Offenbarung, vor und der andere Teil des Treibgases oder Treibgasgemisches liegt als Gas vor. Wird das Auslassventil nun betätigt, treibt der gasförmige Anteil des Treibgases oder Treibgasgemisches, die Wirkstofflösung durch das Ventil nach außen. Jetzt verdampft das in der Zusammensetzung enthaltene Treibgas oder Treibgasgemisch extrem schnell und die zurückbleibende Wirkstofflösung verteilt sich unter Ausbildung eines Schaums sehr fein und gleichmäßig. Als Treibgas für eine Aerosoldose kann vorzugsweise Luft, Kohlendioxid, Distickstoffoxid (N₂O), n-Propan, n-Butan, n-Pentan, Isopentan, Isobutan oder eine beliebige Mischung von diesen eingesetzt werden. Die Menge an Treibgas oder Treibgasgemisch im Spender ist so gewählt, dass sichergestellt werden kann, dass die Dose vollständig entleert werden kann und bis zum Entleeren der Dose immer genügend Treibgas oder Treibgasgemisch in flüssiger und/oder gelöster Form in der Zusammensetzung vorliegt und als Schaumbildner fungieren kann.

Gemäß einer alternativen bevorzugten Ausführungsform ist der Spender eine Bag-on-Valve Dose. Mit dieser Aerosoltechnologie lassen sich auch dickflüssigere / viskosere Produkte austragen. Hierbei werden Treibgas oder Treibgasgemisch und Wirkstofflösung, also die vorstehend beschriebene erfindungsgemäße Zahnpflege- und Zahnreinigungszusammensetzung, voneinander getrennt und liegen in zwei getrennten Kammern des Spenders vor. Ein Beutel, beispielsweise ein Aluminiumbeutel oder ein laminierter Aluminiumbeutel oder ein Beutel aus einem anderen geeigneten Verbundsystem, beinhaltet die Wirkstofflösung und dieser Beutel ist mit dem Ventil verbunden. Ein Treibgas oder Treibgasgemisch, beispielsweise Druckluft, Stickstoff, Kohlendioxid, Distickstoffoxid (N₂O), n-Propan, n-Butan, n-Pentan, Isobutan, Isopentan, ein Propan-Butan-Gemisch oder ein Butan-Pentan-Gemisch, umgibt den Beutel und liefert den notwendigen Druck zum Herausbefördern der Zusammensetzung aus dem Spender. Bei der Betätigung des Auslassventils drückt das Treibgas oder Treibgasgemisch nun Produkt aus dem Beutel. Die äußere Dose besteht vorzugsweise aus Weißblech, Aluminium oder Polyethylenterephthalat, weil diese Materialien stabil, leicht und sehr gut recycelbar sind. Es ist erfindungsgemäß bevorzugt, dass die Bag-on-Valve Dose zur Bevorratung der erfindungsgemäßen Zahnpflege- und Zahnreinigungszusammensetzung einen einzigen Beutel mit der fertigen Zusammensetzung enthält und nicht Teilmischungen der fertigen Zusammensetzung in getrennten Beuteln bevorratet werden und erst bei Betätigung des Abgabeventils miteinander gemischt werden. In einer anderen Ausführungsform werden Teilmischungen der Zusammensetzung in getrennten Beuteln bevorratet und erst bei Betätigung des Abgabeventils miteinander zur fertigen Zusammensetzung gemischt. Diese Form der Bevorratung ermöglicht es, auch nicht miteinander verträgliche Inhaltsstoffe in Kombination einzusetzen, weil diese dann erst unmittelbar vor der Applikation miteinander gemischt werden. Nicht miteinander verträgliche Inhaltsstoffe meint beispielsweise Inhaltsstoffe, die sich bei gemeinsamer Lagerung gegenseitig negativ in ihrer Stabilität beeinflussen wurden oder miteinander reagieren würden.

Gemäß einer besonders bevorzugten Ausführungsform einer Bag-on-Valve Dose bzw. eines Bag-on-Valve Systems befindet sich auch in der erfindungsgemäßen Zusammensetzung ein kleiner Anteil an Treibgas oder einem Treibgasgemisch. Dieser Anteil liegt vorzugsweise in dem Bereich von 0 bis 8 Gew.-%, noch mehr bevorzugt in einem Bereich von 0,5 bis 7 Gew.-% und am bevorzugtesten in einem Bereich von 1,5 bis 6 Gew.-%.

Das Treibgas ist vorzugsweise ausgewählt aus der Gruppe, bestehend aus Isobutan, n-Butan, Isopentan, n-Pentan, Distickstoffoxid (Lachgas; N₂O) oder einer beliebigen Mischung davon.

In einer erfindungsgemäßen Ausführungsform ist das Treibgas Distickstoffoxid (Lachgas). Das Distickstoffoxid wird in einer Menge eingesetzt, dass der resultierende Druck in der Spendervorrichtung vorzugsweise mindestens 4 bar, noch mehr bevorzugt mindestens 5 bar beträgt. Noch mehr bevorzugt liegt der Druck in der Spendervorrichtung im Bereich von 4 bis 10 bar, weiter bevorzugt im Bereich von 6 bis 10 bar.

Distickstoffoxid wird aufgrund seines sehr niedrigen Siedepunkts im Sinne der vorliegenden Offenbarung als eine nicht komprimierbare Flüssigkeit verstanden. Bei Raumtemperatur und einem Druck von 10 bar existiert nur eine Gasphase ohne Flüssiganteile. Im Gegensatz dazu sind beispielsweise Propan, n-Butan, Isobutan, n-Pentan und Isopentan komprimierbare Flüssigkeiten. Diese verflüssigbaren Kohlenwasserstoffe oder Gemische daraus liegen bei Raumtemperatur und einem Druck von 10 bar flüssig mit Gasphase und/oder als Lösung in der Zusammensetzung vor. Komprimierbare Flüssigkeiten im Sinne der vorliegenden Erfindung sind solche, die durch für Aerosolgebinde übliche Kompressionsdrücke, vorzugsweise Drücke im Bereich von 4 bis 10 bar, verflüssigt werden können.

In einer weiteren erfindungsgemäßen Ausführungsform ist das Treibgasgemisch ein Gemisch aus n-Butan, Isobutan, n-Pentan und Isopentan. Vorzugsweise weist das Gemisch einen Anteil von Isopentan von mindestens 70 % und einen Anteil von Isobutan von mindestens 20 % auf. n-Pentan und n-Butan sind jeweils höchstens mit einem Anteil von 5 %, noch mehr bevorzugt von höchstens 3,5 % enthalten. Noch mehr bevorzugt umfasst das Treibgasgemisch 0,1-2 % n-Butan, 22-28 % Isobutan, 0,1-3 % n-Pentan und 72-78 % Isopentan auf. Die Anteile der vier Komponenten sind in dem Gemisch jeweils so gewählt, dass in Summe 100 % für das Treibgasgemisch erreicht werden. Die Einsatzkonzentration eines solchen Gemisches liegt vorzugsweise im Bereich von 2 bis 12 Gew.-%, noch mehr bevorzugt im Bereich von 3 bis 10 Gew.-%.

Das Treibgas oder das Treibgasgemisch ist gemäß einer weiteren, bevorzugten Ausführungsform der vorliegenden Erfindung so ausgewählt oder zusammengesetzt, dass es bei Normaldruck (1,013 hPa) einen Siedepunkt von -10 °C oder höher, noch mehr bevorzugt von -8 °C oder höher aufweist. Gemäß einer Ausführungsform der Erfindung ist das Treibgas n-Butan. Vorzugsweise liegt die Konzentration von n-Butan, bezogen auf die Zusammensetzung ohne Treibgas, also die reine Flüssigformulierung, im Bereich von 2 bis 12 Gew.-%, noch mehr bevorzugt im Bereich von 3 bis 10 Gew.-%.

Das in der erfindungsgemäßen Zusammensetzung enthaltene Treibgas oder Treibgasgemisch fungiert als Schaumbilder.

Auch für die Ausführungsform der Aerosoldose werden für die Herstellung eines Schaums oder Aerosols als Treibgas vorzugsweise n-Propan, n-Butan, n-Pentan, Isobutan, Isopentan oder beliebige Mischungen davon verwandt. Alternativ können auch Luft, Kohlendioxid, Distickstoffoxid oder beliebige Mischungen davon als Treibmittel eingesetzt werden. Es ist wiederum besonders bevorzugt, dass das Treibmittel oder das Treibmittelgemisch so ausgewählt, dass es keine Verbindung enthält, die bei Normaldruck einen Siedepunkt von weniger als -10 °C, noch mehr bevorzugt von weniger als -8 °C aufweist.

Auch für die Aus führungs form der Aerosoldose ist das Treibgas in einer erfindungsgemäßen Ausführungsform Distickstoffoxid (Lachgas). Das Distickstoffoxid wird in einer Menge eingesetzt, dass der resultierende Druck in der Spendervorrichtung vorzugsweise mindestens 4 bar, noch mehr bevorzugt mindestens 5 bar beträgt. Noch mehr bevorzugt liegt der Druck in der Spendervorrichtung im Bereich von 4 bis 10 bar, weiter bevorzugt im Bereich von 6 bis 10 bar.

In einer weiteren erfindungsgemäßen Ausführungsform ist das Treibgasgemisch in der Aerosoldose ein Gemisch aus n-Butan, Isobutan, n-Pentan und Isopentan. Vorzugsweise weist das Gemisch einen Anteil von Isopentan von mindestens 70 % und einen Anteil von Isobutan von mindestens 20 % auf. n-Pentan und n-Butan sind jeweils höchstens mit einem Anteil von 5 %, noch mehr bevorzugt von höchstens 3,5 % enthalten. Noch mehr bevorzugt umfasst das Treibgasgemisch 0,1-2 % n-Butan, 22-28 % Isobutan, 0,1-3 % n-Pentan und 72-78 % Isopentan auf. Die Anteile der vier Komponenten sind in dem Gemisch jeweils so gewählt, dass in Summe 100 % für das Treibgasgemisch erreicht werden. Die Einsatzkonzentration eines solchen Gemisches liegt vorzugsweise im Bereich von 2 bis 12 Gew.-%, noch mehr bevorzugt im Bereich von 3 bis 10 Gew.-%, bezogen auf die Zusammensetzung ohne Treibgas, also die reine Flüssigformulierung.

Das Treibgas oder das Treibgasgemisch der Aerosoldose ist gemäß einer weiteren, bevorzugten Ausführungsform der vorliegenden Erfindung so ausgewählt oder zusammengesetzt, dass es bei Normaldruck (1,013 hPa) einen Siedepunkt von -10 °C oder höher, noch mehr bevorzugt von -8 °C oder höher aufweist. Gemäß einer Ausführungsform der Erfindung ist das Treibgas n-Butan. Vorzugsweise liegt die Konzentration von n-Butan, bezogen auf die Zusammensetzung ohne Treibgas, also die reine Flüssigformulierung, im Bereich von 2 bis 12 Gew.-%, noch mehr bevorzugt im Bereich von 3 bis 10 Gew.-%.

Eine Alternative zur Herstellung eines Schaums oder eines Aerosols aus der Zusammensetzung stellt die Verwendung eines manuellen oder elektrischen Pumpsystems dar. Wird die Pumpe betätigt, entsteht ein Überdruck zwischen einem äußerem und einem inneren Behälter, der nach Betätigung des Ventils das Aerosol bzw. den Schaum bildet. Die Anwendung der Zusammensetzung als Schaum ist besonders bevorzugt.

In einem weiteren Aspekt betrifft die vorliegende Erfindung die Verwendung des vorstehend beschriebenen Systems zur Bevorratung und Abgabe der erfindungsgemäßen Zahnpflege- und Zahnreinigungszusammensetzung. Dabei wird die Zusammensetzung vorzugsweise als zunächst nur wenig aufgeschäumter Strang auf den oder die Mundeinsatzelemente der Zahnreinigungsvorrichtung aufgebracht. Nach der Applikation weist der Strang bei 20 °C und Normaldruck vorzugsweise eine Expansionsrate von mehr als 100 Vol.-% in 3 bis 5 Sekunden auf. Noch mehr bevorzugt liegt die Expansionsrate bei 20 °C und Normaldruck nach 3 bis 5 Sekunden in einem Bereich von 150 Vol.-% bis 1000 Vol.-%. Der so erhaltene Schaum wird dann zur Reinigung von Zahnoberflächen, Zahnzwischenräumen und der Mundhöhle verwendet.

In einem noch weiteren Aspekt betrifft die vorliegende Erfindung ein Verfahren zum gleichzeitigen Reinigen mehrerer, vorzugsweise aller Zähne mittels einer Vibrationszahnputzvorrichtung für die gleichzeitige Reinigung mehrerer, vorzugsweise aller Zähne eines Benutzers, wobei die Zusammensetzung dargereicht wird durch Aufbringen der Zusammensetzung auf einen Mundeinsatz oder mehrere Mundeinsätze der Zahnputzvorrichtung. Die Zahnreinigung dient insbesondere zum Vorbeugen und/oder Behandeln von Erkrankungen der Zähne, der Zahnhälse und/oder des Zahnfleisches.

Das Verfahren gemäß der vorliegenden Offenbarung umfasst entsprechend die folgenden Schritte:
- Bereitstellen einer thixotropen Zahnpflege- und Zahnreinigungszusammensetzung,
- Auftragen/Aufbringen der thixotropen Zusammensetzung auf einen Mundeinsatz oder mehrere Mundeinsätze, der bzw. die für die Reinigung mehrerer, bevorzugt aller Zähne eines Benutzers geeignet ist/sind,
- Einführen des Mundeinsatzes bzw. der Mundeinsätze mit der Zusammensetzung in den Mund des Benutzers und
- Reinigen mehrerer, bevorzugt aller Zähne des Benutzers mit dem/den Mundeinsatz/ Mundeinsätzen und der Zusammensetzung. Das Reinigen erfolgt vorzugsweise dadurch, dass der oder die Mundeinsätze mittels einer Antriebsvorrichtung zur Vibration angeregt werden.

Für das erfindungsgemäße Verfahren ist die hierin beschriebene erfindungsgemäße Zahnpflege- und Zahnreinigungszusammensetzung besonders geeignet. Sie stellt nicht nur eine ausreichende Wirkstoffmenge zur Verfügung, sondern kann darüber hinaus besonders gleichmäßig und einfach auf dem Mundeinsatz oder den Mundeinsätzen verteilt werden. Auch wird eine Überdosierung, wie sie bei herkömmlichen Zahncremes, -pasten oder -gelen auftreten würde, vermieden.

Die bereitgestellte Zusammensetzung ist somit vorzugsweise die erfindungsgemäße Zahnpflege- und Zahnreinigungszusammensetzung, umfassend
a) 0,5 bis 30 Gew.-%, vorzugsweise 1 bis 30 Gew.-%, Putzkörper,
b) 0,5 bis 5 Gew.-% wenigstens eines Schichtsilikats,
c) 0,1 bis 2 Gew.-% wenigstens eines pyrogenen Silikats, vorzugsweise eines hydrophoben pyrogenen Silikats
d) 0,2 bis 5 Gew.-% wenigstens einer oberflächenaktiven Substanz, und
e) ein Lösungsmittel, vorzugsweise Wasser, ad 100 Gew.-%.

Bezüglich der bevorzugten wesentlichen und optionalen Inhalts- und Hilfs- oder Zusatzstoffe sowie deren bevorzugter Anteilsbereiche wird ausdrücklich auf die vorstehenden Ausführungen im Zusammenhang mit der Zusammensetzung als solches Bezug genommen.

Das Aufbringen/Auftragen der Zusammensetzung auf den Mundeinsatz oder die Mundeinsätze erfolgt vorzugsweise als Schaum, vorzugsweise als Schaumspray, oder als Aerosol. Die Zusammensetzung kann dann auf dem Mundeinsatz oder den Mundeinsätzen noch weiter expandieren und ein Zahnreinigungsmousse ausbilden. Vorzugsweise weist die zunächst leicht aufgeschäumte Zahnpflege- und Zahnreinigungszusammensetzung nach Applikation bei 20 °C und Normaldruck eine Expansionsrate von mehr als 100 Vol.-% in 3 bis 5 Sekunden auf. Noch mehr bevorzugt liegt die Expansionsrate bei 20 °C und Normaldruck nach 3 bis 5 Sekunden in einem Bereich von 150 Vol.-% bis 1000 Vol.-%.

Durch diese Applikation der Zusammensetzung auf den Mundeinsatz oder die Mundeinsätze wird die gleichmäßige und sparsame Verteilung der Zusammensetzung für den Benutzer weiter erleichtert und es kann eine gute Reinigung und Pflege der Zähne und des Zahnfleisches sichergestellt werden. Zudem weist ein Aerosol oder Schaum (direkt oder als Schaumspray) oder das Mousse eine gewisse Lebensdauer auf, bevor er sich in seine Grundzusammensetzung auflöst. In dieser Zeit kann der Mundeinsatz bzw. können die Mundeinsätze in den Mund des Benutzers eingeführt werden. Der auf den Mundeinsatz aufgetragene Schaum / Aerosol oder das Mousse wird dann durch den Raum zwischen den Zähnen und Mundeinsatz zerdrückt und gleichmäßig verteilt. Somit kann der Schaum oder das Aerosol sehr sparsam auf den Mundeinsatz oder die Mundeinsätze aufgetragen werden.

Das Aufbringen der Zusammensetzung erfolgt vorzugsweise mit Hilfe eines der hierin beschriebenen Spender durch den die erfindungsgemäße Zusammensetzung in einen Schaum, vorzugsweise ein Schaumspray, oder ein Aerosol überführt werden kann. Bezüglich bevorzugter Ausführungen für diesen Spender wird ausdrücklich auf die vorstehende Offenbarung verwiesen.

Das erfindungsgemäße Zahnreinigungsverfahren umfasst weiterhin das Einführen des Mundeinsatzes oder der Mundeinsätze mit der Zusammensetzung in den Mund eines Benutzers und das gleichzeitige Reinigen und Pflegen mehrerer, bevorzugt aller Zähne des Benutzers durch Vibration des Mundeinsatzes. Die exakten Ausführungen der Mundreinigung über unterschiedliche Reinigungsstrukturen an den Innenflächen des Mundeinsatzes, Vibrationsfrequenzen, Reinigungsprogramme und Anwendungsdauer sind in der vorliegenden Erfindung nicht beschränkt.

Ein erfindungsgemäß zur Zahnreinigung und -pflege verwendbarer Mundeinsatz kann zur Reinigung aller Zähne zwei U-förmige Wannen zur Aufnahme der Zähne aufweisen, wobei sich die beiden Wannen dem Gebiss des Benutzers passen und in entgegengesetztem Richtungen geöffnet sind. Würde eine zu niedrigviskose Zahnreinigungszusammensetzung verwendet werden, kann diese beim Einsetzen des Mundeinsatzes aus der unteren Wanne herausfließen oder sich am Außenrand der unteren Wanne sammeln, was das Reinigungsergebnis, insbesondere an den Kauflächen der Zähne des Unterkiefers, negativ beeinträchtigen kann. Ein Aerosol, Schaum oder Mousse verbleibt hingegen durch seine inhärente Festigkeit längere Zeit in der Wanne, auch wenn diese mit der Öffnung nach unten gedreht wird.

In einer bevorzugten erfindungsgemäßen Ausführungsform wird der Mundeinsatz bzw. werden die Mundeinsätze zum Reinigen der Zähne, der Zahnhälse und des Zahnfleisches im Mund des Benutzers durch einen Motor zu Schwingungen/ Vibrationen mit einer Frequenz im Bereich von 20 bis 700 Hz, bevorzugt 25 bis 600 Hz angeregt.

Das gleichzeitige Reinigen der mehreren, vorzugsweise aller Zähne des Benutzers, dauert vorzugsweise zwischen 2 Sekunden und 5 Minuten, noch bevorzugter liegt die Zahnputzdauer in einem Bereich von 3 Sekunden bis zu 4 Minuten. Alternativ liegt die Obergrenze der Putzdauer bei 2 Minuten, 1,5 Minuten oder 1 Minute.

In einer erfindungsgemäßen Ausführungsform weist der Mundeinsatz eine Form auf, die die Zähne eines Benutzers während der Benutzung umgibt, so dass während des Reinigungsvorgangs Kauflächen und Innen- und Außenflanken der Zähne jeweils einer Fläche des Mundeinsatzes gegenüberliegen, und wobei eine Vielzahl von Reinigungsstrukturen auf den Zähnen zugewandten Seiten des Mundeinsatzes vorgesehen sind.

In einer erfindungsgemäßen Ausführungsform ist der Mundeinsatz individuell an die Gebissform des Benutzers angepasst. Diese Anpassung kann durch herkömmliche negativ-positiv-Abdrücke, Abscannen und anschließendes 3D-Drucken des Mundeinsatzes, oder andere geeignete Verfahren erfolgen. Bevorzugte Materialien für den Mundeinsatz sind biokompatible Kunststoffe, z.B. biokompatibles Polyamid.

In einer erfindungsgemäßen Ausführungsform ist der Mundeinsatz einteilig oder, jeweils für den Oberkiefer und den Unterkiefer, zweiteilig ausgebildet.

Die Fig. 1 zeigt eine mögliche Ausführungsform eines Mundeinsatzes zur Anwendung mit einer Zusammensetzung gemäß einer bevorzugten Ausführungsform der vorliegenden Offenbarung. Insbesondere zeigt Fig. 1 eine perspektivische Ansicht eines einteiligen Mundeinsatzes 10. Die nicht sichtbare untere Struktur des Mundeinsatzes ist der oberen im Wesentlichen identisch, wobei anpassungsbedingte Formabweichungen möglich sind. Die Wandstärke der Wände 12 des Mundeinsatzes 10 beträgt typischerweise 1 bis 2,5 mm. Die äußere Begrenzung der Wände 12 verläuft in der Regel 1 bis 3 mm oberhalb des Zahnfleischrandes. Der Abstand zwischen den Reinigungsflächen, d.h. denjenigen Flächen des Mundeinsatzes 10, die beim Reinigungsvorgang den Zahnflächen des Benutzers zugewandt sind, und den Zahnoberflächen beträgt 0 bis 5 mm in Abhängigkeit von den gewählten Reinigungsstrukturen 14. In einer bevorzugten Ausführungsform sind auf den Reinigungsflächen Reinigungsstrukturen 14 vorgesehen.

An dem Mundeinsatz 10 der Ausführungsform in Fig.1 ist ein Kupplungselement 16 zur Verbindung des Mundeinsatzes 10 mit einer Antriebsvorrichtung (nicht gezeigt) angebracht. Die Antriebsvorrichtung ist grundsätzlich mit Antriebsvorrichtungen vergleichbar, wie sie aus dem Stand der Technik auf dem Gebiet der Schallzahnbürsten oder dergleichen bekannt sind. Die Kopplung mit der Antriebsvorrichtung erfolgt über eine passende Bohrung 24.

In Fig. 1 wird verdeutlicht, dass ein relativ genau an das Gebiss des Benutzers angepasster Mundeinsatz viele Ausbuchtungen (den Zähnen entsprechend) und Stege (den Zahnzwischenräumen und Kauflächen entsprechend) aufweist. Diese Unebenheiten erschweren die Nutzung mit einer herkömmlichen Zahncreme oder ähnlichen Präparaten deutlich. Entweder muss sehr viel Zahncreme aufgetragen werden, was die Anwendungskosten erhöht und Ressourcen verschwendet. Ein akkurates Verteilen einer zähflüssigen Zahncreme mit einer Bürste oder einem Spatel wäre außerdem sehr zeitaufwändig für den Anwender/Benutzer. Die Reinigungsstrukturen 14 (in Fig. 2 gezeigt und unten detailliert beschrieben) erzeugen zusätzlich feine Unebenheiten, die mit einer herkömmlichen Zahncreme kaum zu erreichen bzw. auszufüllen sind.

Fig. 2 zeigt eine Detailansicht von Reinigungsstrukturen 14 in Form von Reinigungselementen, die an der Oberfläche des Mundeinsatzes 10 aus Fig. 1 vorgesehen sein können. In diesem Fall handelt es sich jeweils um Reinigungselemente, die einstückig mit dem Mundeinsatz 10 an dessen Oberfläche gebildet sind. Im Bereich der Kauflächen sind in dieser Ausführungsform rautenförmige Reinigungselemente 28 vorgesehen, im Bereich der vorderen und hinteren Zahnflächen sind jeweils zylinderförmige Reinigungselemente 30 ausgebildet. Aufgrund der Rautenform sind die Reinigungselemente 28 härter als die zylinderförmigen Reinigungselemente 30, so dass im Bereich der Kauflächen eine intensivere Zahnputzwirkung erzielt wird. Selbstverständlich können die Reinigungselemente 30 an den vorderen und hinteren Zahnflächen verschieden ausgebildet sein. Ebenso könnten in jedem der in Fig. 2 gezeigten Bereiche, d. h. im Bereich der hinteren Zahnflächen, der Kauflächen und der vorderen Zahnflächen, auch andere Reinigungsstrukturen 14 vorgesehen sein, beispielsweise Gummierungsschichten und/oder Streifenbürsten.

Durch die variierende Formgebung der Reinigungsstrukturen wird die Nutzung herkömmlicher Zahncremes zusätzlich erschwert. Dringt eine bestimmte Zahncreme beispielsweise gut in den Raum zwischen den rautenförmigen Elementen 28 ein, so sind die Abstände zwischen den Borstenstrukturen 30 zu gering, um eine ausreichende und einfache Verteilung der Zahncreme zu ermöglichen und bei unachtsamer Anwendung kann sich das Reinigungsergebnis verschlechtern.

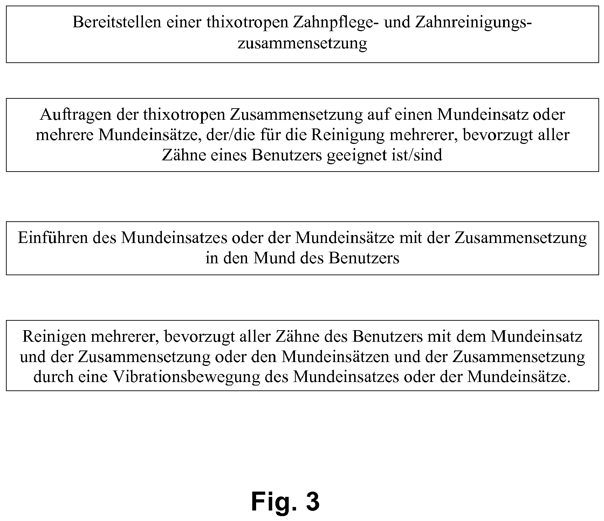

Das durch die vorliegende Offenbarung bereitgestellte Verfahren zum Reinigen und Pflegen der Zähne und/oder des Zahnfleisches eines Benutzers, insbesondere zum Vorbeugen und/oder Behandeln von Erkrankungen der Zähne, der Zahnhälse und/oder des Zahnfleisches mit einer thixotropen Mund-, Zahnpflege- und Zahnreinigungszusammensetzung wird in Fig. 3 zusammengefasst.

Das erfindungsgemäße Verfahren zum Reinigen und Pflegen der Zähne und/oder des Zahnfleisches sollte vorzugsweise mindestens zweimal am Tag, noch mehr bevorzugt zwei bis viermal pro Tag, beispielsweise zweimal oder dreimal pro Tag, durchgeführt werden.

Gemäß eines weiteren Aspekts betrifft die vorliegende Erfindung eine thixotrope Zahnpflege- und Zahnreinigungszusammensetzung zur Verwendung in einem Verfahren zum Vorbeugen und/oder Behandeln von Erkrankungen der Zähne oder Zahnhälse, insbesondere von Karies, Parodontose, Plaque, sowie von Erkrankungen des Zahnfleisches mittels einer Zahnputzvorrichtung für die gleichzeitige Reinigung mehrerer, vorzugsweise aller Zähne eines Benutzers, wobei die Zusammensetzung dargereicht wird durch Aufbringen der Zusammensetzung auf einen Mundeinsatz oder mehrere Mundeinsätze der Zahnputzvorrichtung.

Dabei umfasst die thixotrope Zahnpflege- und Zahnreinigungszusammensetzung
a) 0,5 bis 30 Gew.-%, vorzugsweise 1 bis 30 Gew.-%, Putzkörper,
b) 0,5 bis 5 Gew.-% wenigstens eines Schichtsilikats,
c) 0,1 bis 2 Gew.-% wenigstens eines pyrogenen Silikats, vorzugsweise wenigstens eines hydrophoben pyrogenen Silikats,
d) 0,2 bis 5 Gew.-% wenigstens einer oberflächenaktiven Substanz, und
e) ein Lösungsmittel, vorzugsweise Wasser, ad 100 Gew.-%.

Bevorzugte Ausführungsformen des Verfahrens und der Verwendung sind oben im Zusammenhang mit der Offenbarung zu der Zusammensetzung beschrieben. Da diese identisch auf das Verfahren und die Verwendung der Zusammensetzung übertragen werden können, wird hierauf Bezug genommen.

Die Anwendung einer thixotropen Zusammensetzung gemäß der vorliegenden Offenbarung ermöglicht das leichte und fehlerfreie Auftragen auf einen Mundeinsatz einer Zahnputzvorrichtung, insbesondere auf einen Mundeinsatz, der zum gleichzeitigen Reinigen mehrerer Zähne oder aller Zähne des Ober- und/oder Unterkiefers ausgebildet ist. Durch das bevorzugte Auftragen der Zusammensetzung in Form eines Schaums, noch mehr bevorzugt in Form eines Schaumsprays, wird insbesondere eine verbesserte Haftung auf den Reinigungsflächen des in Fig. 1 gezeigten Mundeinsatzes gewährleistet, die nicht gezeigt, also nach unten gerichtet sind.

Ebenso betrifft die vorliegende Erfindung die Verwendung der hierin beschriebenen Zusammensetzung zur Verwendung im Bereich der Mund- und Zahnhygiene. Bevorzugt wird die Zusammensetzung mittels einer Zahnputzvorrichtung für die gleichzeitige Reinigung mehrerer, vorzugsweise aller Zähne eines Benutzers appliziert. Die Zusammensetzung wird gemäß dieser bevorzugten Ausführungsform dargereicht durch Aufbringen der Zusammensetzung auf einen Mundeinsatz oder mehrere Mundeinsätze der Zahnputzvorrichtung.

### Messmethoden:

Die Bestimmung der Viskosität erfolgt mit einem hochpräzisen Rotationsviskosimeter mit integrierter Dichtemesszelle (SVM™ 3001 Stabinger Viskosimeter™ von Anton Paar) gemäß der Norm ASTM D 7042. Das Anton Paar Viskosimeter SVM 3001 arbeitet nach dem Stabinger Messprinzip. Es basiert auf dem Couette-Prinzip, mit integrierter Dichtemesszelle. In einem einzigen Messzyklus können die dynamische Viskosität, Dichte, kinematische Viskosität und der Viskositätsindex bestimmt werden. Ein mit konstanter Drehzahl rotierendes Rohr wird mit der Probe gefüllt, in welcher sich der Messrotor, der langsamer als das Außenrohr rotiert, befindet. Es werden Drehmoment und Drehzahl bestimmt.

Das SVM™ 3001 Stabinger Viskosimeter™ von Anton Paar basiert auf dem Couette-Prinzip, mit integrierter Dichtemesszelle. In einem einzigen Messzyklus können die dynamische Viskosität, Dichte, kinematische Viskosität und der Viskositätsindex bestimmt werden. Die Messzellenkombination deckt den gesamten Messbereich für Viskosität, Dichte und Temperatur ab. Der Messbereich für Viskosität beträgt 0,2 bis 30 000 mm²/s; die simultane Dichte-Bestimmung erfolgt gemäß ASTM D4052 und ISO 12185. Es kann in einem Temperaturbereich von -60 ° bis +135 ° C betrieben werden.

Die Messungen erfolgen im newtonschen Bereich der Proben. Falls nicht anderes angegeben, gelten alle Viskositätsangaben bei 20 °C und Normaldruck von 0,1 MPa (1013,25 mbar). Bestimmt wird die Viskosität der flüssigen Zusammensetzung, also zu einem Zeitpunkt bevor die Zusammensetzung gemäß einer bevorzugten Ausführungsform in einen Schaum oder ein Aerosol überführt wird.

Die Bestimmung der spezifischen Oberfläche in (m²/g) nach der BET-Methode durch die Adsorption von Gasen, beispielsweise von Stickstoff N₂, erfolgt nach Brunauer, Emmett und Teller gemäß DIN 66131.

Die Bestimmung der mittleren Teilchengröße der Silikate erfolgt mittels Laserbeugung gemäß ISO 13320. Bestimmt wird der d50-Wert.

Die Bestimmung des RDA-Werts erfolgt nach nach der in dem Artikel "A Laboratory Method for Assessment of Dentifrice Abrasivity", John J. Hefferren, im Journal of Dental Research, Vol. 55, Nr. 4 (1976), Seiten 563-573 beschriebenen Methode.

Der Messing-Einlehner-(BE)-Abrasionswert bzw. die Einlehner Abrasion wurde durch die Verwendung einer Einlehner AT-1000 Abriebprüfmaschine gemessen. In dieser Prüfung wird ein Fourdrinier-Messingdrahtsieb gewogen und der Wirkung einer 10%-igen wässerigen Silikatsuspension für eine bestimmte Anzahl an Umdrehungen ausgesetzt und die Abriebmenge dann als Milligramm Materialverlust von dem Fourdrinier-Drahtsieb pro 100.000 Umdrehungen bestimmt. Das Ergebnis ist der 10% Messing-Einlehner-(BE)-Abrasionswert.

Der Drehkörper der Abriebprüfmaschine läuft unabhängig von der Netzfrequenz (50/60 Hz) einheitlich mit 1800 Umdrehungen/Minute. Die Silikatsuspension wird durch Mischen des zu testenden Silikats mit entionisiertem Wasser hergestellt. Zur Bereitstellung einer 10%igen Suspension werden beispielsweise 100 g Silikat mit 900 g Wasser gemischt.

### BEISPIELE

### Beispiel 1 (Zahnpflegezusammensetzung)

| | |
|---|---|
| Sorbosil AC 77: | 6 Gew.-% |
| Sorbosil AC 35: | 3 Gew.-% |
| Sorbosil TC 15: | 1 Gew.-% |
| Pyrogenes Silikat: | 0,5 Gew.-% |
| Schichtsilikat, vorzugsweise Laponite: | 2,5 Gew.-% |
| Kaliumchlorid: | 0,13 Gew.-% |
| Calciumhydroxylapatit: | 0,47 Gew.-% |
| Xylitol: | 10,0 Gew.-% |
| d-Panthenol: | 0,6 Gew.-% |
| Coco Glucoside: | 0,5 Gew.-% |
| Natriummethylparaben: | 0,17 Gew.-% |
| Xanthan Gum: | 0,05 Gew.-% |
| Acrylate/C10-30-Alkyl-Acrylat-Crosspolymer: | 0,05 Gew.-% |
| Aroma: | 0,2 Gew.-% |
| Aqua osm.: | ad 100 Gew.-% |

### Beispiel 2 (Zahnpflegezusammensetzung)

| | |
|---|---|
| Sorbosil AC 77: | 6 Gew.-% |
| Sorbosil AC 35: | 3 Gew.-% |
| Sorbosil TC 15: | 1 Gew.-% |
| Pyrogenes Silikat: | 0,5 Gew.-% |
| Schichtsilikat, vorzugsweise Laponite: | 2,5 Gew.-% |
| Kaliumchlorid: | 0,13 Gew.-% |
| Aminfluorid: | 0,3 Gew.-% |
| Xylitol: | 10,0 Gew.-% |
| d-Panthenol: | 0,6 Gew.-% |
| Coco Glucoside: | 0,5 Gew.-% |
| Natriummethylparaben: | 0,17 Gew.-% |
| Aroma: | 0,2 Gew.-% |
| Aqua osm.: | ad 100 Gew.-% |

Die durch übliches Zusammenfügen der einzelnen Bestandteile erhaltenen Zusammensetzungen wurden jeweils in Bag-on-Valve Dosen abgefüllt. Während des Abfüllprozesses in den jeweiligen Beutel der Bag-on-Valve Dose wurden den Zusammensetzungen jeweils noch 4,9 Gew.-% kosmetischer Schaumbildner (Treibgas), bezogen auf die treibgashaltige Zusammensetzung, hinzugefügt.

Die vorstehend genannten, beispielhaften Zusammensetzungen können in den Mundstücken manuell durch Aufsprühen als Schaum oder Aerosol in die Mundstücke verteilt werden. Sie sind dadurch sehr gleichmäßig auf den Mundstücken verteilt. Die Zusammensetzungen haften gut an den Mundstücken. Die Anhaftung (Adhäsion) ist messbar mit einem Tensiometer.

Es hat sich gezeigt, dass insbesondere der Putzkörper, aber auch die anderen Aktivstoffe gleichmäßig in dem Schaum oder Aerosol verteilt sind. Damit ist sichergestellt, dass lokale Überdosierungen oder Unterdosierungen vermieden werden.

## Patentansprüche

1. Thixotrope Zahnpflege- und Zahnreinigungszusammensetzung, umfassend
a) 0,5 bis 30 Gew.-% Putzkörper, vorzugsweise wenigstens eines gefällten Silikats
b) 0,5 bis 5 Gew.-% wenigstens eines Schichtsilikats,
c) 0,1 bis 2 Gew.-% wenigstens eines pyrogenen Silikats,
d) 0,2 bis 5 Gew.-% wenigstens einer oberflächenaktiven Substanz, und
e) Lösungsmittel, vorzugsweise Wasser, ad 100 Gew.-%.

2. Zusammensetzung nach Anspruch 1, wobei der Putzkörper in eine thixotrope Matrix umfassend die Komponenten b) bis e) eingebettet ist.

3. Zusammensetzung nach Anspruch 1 oder Anspruch 2, wobei das wenigstens eine Schichtsilikat ein künstliches Schichtsilikat ist, das scheibenförmige Mineralpartikeln mit einer mittleren Dicke von vorzugsweise 0,1 bis 5 nm und mit einem mittleren Durchmesser im Bereich von 10 bis 50 nm umfasst oder aus diesen besteht.

4. Zusammensetzung nach Anspruch 3, wobei das Schichtsilikat Laponite ist.

5. Zusammensetzung nach einem der vorhergehenden Ansprüche, wobei das wenigstens eine pyrogene Silikat eine spezifische Oberfläche (BET) im Bereich von 100 bis 300 m²/g, vorzugsweise im Bereich von 150 bis 250 m²/g aufweist und noch mehr bevorzugt ein hydrophobes pyrogenes Silikat ist, das eine spezifische Oberfläche (BET) im Bereich von 100 bis 300 m²/g, vorzugsweise im Bereich von 150 bis 250 m²/g aufweist.

6. Zusammensetzung nach einem der vorhergehenden Ansprüche, wobei die wenigstens eine oberflächenaktive Substanz ein nichtionisches Tensid, vorzugsweise ein C₆-C₂₀-Alkylglykosid, oder eine Mischung nichtionischer Tenside, vorzugsweise eine Mischung von C₆-C₂₀-Alkylglykosiden, umfasst oder ist.

7. Zusammensetzung nach einem der vorhergehenden Ansprüche, wobei die Zusammensetzung als Feuchthaltemittel, vorzugsweise als einziges Feuchthaltemittel, Xylitol, vorzugsweise mit einem Anteil von weniger als 20 Gew.-%, umfasst, und die Zusammensetzung vorzugsweise im Wesentlichen frei von Sorbitol, Glycerin, 1,2-Propylenglycol und beliebigen Mischungen davon ist.

8. Zusammensetzung nach einem der vorhergehenden Ansprüche, wobei die Zusammensetzung weiterhin wenigstens einen Wirkstoff umfasst, der ausgewählt ist aus der Gruppe, bestehend aus einer oder mehrerer Verbindungen zur Remineralisation oder Keramisierung, vorzugsweise Hydroxylapatit, einem oder mehrerer Fluoridwirkstoff, einem Mittel zur Zahnfleischpflege, einem desinfizierenden Bestandteil und einer beliebigen Mischung davon.

9. Zusammensetzung nach einem der vorhergehenden Ansprüche, wobei die Zusammensetzung übliche Hilfs- und Zusatzstoffe ausgewählt aus der Gruppe, bestehend aus Aromastoffe, Süßungsmittel, Konservierungsmittel, Pigmente, Farbstoffe, Puffersubstanzen und einer beliebigen Mischung davon umfasst.

10. Zusammensetzung nach einem der vorhergehenden Ansprüche, wobei die Zusammensetzung mindestens ein Silikat aus der Gruppe der gefällten Silikate, vorzugsweise mindestens ein gefälltes Silikat mit einer mittleren Teilchengröße in einem Bereich von mehr als 12 bis 20 µm, mindestens ein pyrogenes Silikat und mindestens ein Schichtsilikat umfasst, wobei die Zusammensetzung vorzugsweise im Wesentlichen frei von Viskositätsverstärker ausgewählt aus der Gruppe, bestehend aus Polysacchariden, Polymeren und beliebigen Mischungen davon ist.

11. Zusammensetzung nach einem der vorhergehenden Ansprüche, wobei die Zusammensetzung einen Thixotropie-Index im Bereich von 1 bis 10 aufweist.

12. Zusammensetzung nach einem der vorhergehenden Ansprüche, wobei die Zusammensetzung als Schaum oder Aerosol vorliegt.

13. System, bestehend aus einer Zahnpflege- und Zahnreinigungszusammensetzung nach einem der vorhergehenden Ansprüche 1 bis 11 und einem Spender mit Mitteln zur Bevorratung, zur Förderung und Abgabe der Zahnpflege-und Zahnreinigungszusammensetzung,
wobei das Mittel zur Abgabe der Zusammensetzung ein Abgabeventil mit einer Abgabeöffnung aufweist, und
wobei die Zusammensetzung und der Spender derart zusammenwirken, dass bei Betätigung des Abgabeventils das Zahnreinigungsmittel, vorzugsweise in strangförmiger und/oder vorzugsweise wenig aufgeschäumter Form, aus der Ausgabeöffnung austritt.

14. System nach Anspruch 13, wobei die Zahnpflege- und Zahnreinigungszusammensetzung in Kombination mit einem Treibgas oder Treibgasgemisch vorliegt.

15. System nach Anspruch 13 oder Anspruch 14, wobei das Mittel zur Förderung die Zusammensetzung mittels eines Treibgases oder Treibgasgemisches befördert und der Spender ausreichend druckstabil gegenüber dem Expansionsdruck der treibgashaltigen Zusammensetzung oder des Treibgases oder Treibgasgemisches ausgebildet sind.

16. System nach einem der Ansprüche 13 bis 15, wobei das Mittel zur Bevorratung und Angabe der Zusammensetzung eine Aerosoldose oder eine Bag-on-Valve- oder eine Kolbendose ist.

17. System nach einem der Ansprüche 13 bis 16, wobei das Zahnreinigungsmittel ein Treibgas oder Treibgasgemisch enthält, das eine komprimierbare Flüssigkeit ist, die bei Normaldruck einen Siedepunkt von -10 °C oder höher aufweist, oder wobei das Treibgas oder Treibgasgemisch nicht als komprimierte Flüssigkeit vorliegt, und vorzugsweise Distickstoffoxid ist.

18. System nach einem der Ansprüche 13 bis 17, wobei der Treibgasgehalt der Zusammensetzung 0 bis 15 Gew.-%, vorzugsweise kleiner gleich 12 Gew.-%, beträgt, oder im Fall der Verwendung einer nicht komprimierbaren Flüssigkeit, vorzugsweise von Distickstoffoxid, als Treibgas oder in einem Treibgasgemisch der resultierende Druck im Bereich von 4-10 bar, vorzugsweise im Bereich von 6-10 bar liegt.

19. Verfahren zum gleichzeitigen Reinigen mehrerer, vorzugsweise aller Zähne eines Subjekts, wobei das Zahnreinigungsverfahren die folgenden Schritte umfasst:
- Bereitstellen einer thixotropen Zahnpflege- und Zahnreinigungszusammensetzung, vorzugsweise einer thixotropen Zahnpflege- und Zahnreinigungszusammensetzung gemäß einem der Ansprüche 1 bis 12,
- Auftragen/Aufbringen der thixotropen Zusammensetzung auf einen Mundeinsatz oder mehrere Mundeinsätze einer Vibrationszahnputzvorrichtung für die gleichzeitige Reinigung mehrerer, vorzugsweise aller Zähne eines Benutzers, der bzw. die für die Reinigung mehrerer, bevorzugt aller Zähne eines Benutzers geeignet ist/sind,
- Einführen des Mundeinsatzes bzw. der Mundeinsätze mit der Zusammensetzung in den Mund des Benutzers und
- Reinigen mehrerer, bevorzugt aller Zähne des Benutzers mit dem/den Mundeinsatz/ Mundeinsätzen und der Zusammensetzung durch Aktivieren der Vibrationszahnputzvorrichtung.

20. Zahnpflege- und Zahnreinigungszusammensetzung nach einem der Ansprüche 1 bis 12 zur Verwendung in einem Verfahren zur Vorbeugung und/oder Behandlung von Zahn- und Zahnfleischerkrankungen, insbesondere von Karies, Parodontose und Plaque.
